# EUROPEAN PATENT APPLICATION

(11) **EP 4 265 217 A1**
(43) Date of publication of application: **25.10.2023**
(21) Application number: 21904938.4
(22) Date of filing: 10.05.2021
(51) Int. Cl.: A61B 34/37, A61B 17/00

(54) **SURGICAL INSTRUMENT, SLAVE OPERATING EQUIPMENT, AND SURGICAL ROBOT**

(30) Priority: 19.12.2020 CN 202011512462
(71) Applicant: Shenzhen Edge Medical Co., Ltd., Shenzhen, Guangdong 518000 (CN)
(72) Inventor: WANG, Jianchen, Shenzhen, Guangdong (CN); GAO, Yuanqian, Shenzhen, Guangdong (CN)
(74) Representative: Viering, Jentschura & Partner mbB Patent- und Rechtsanwälte
(86) International application number: PCT/CN2021/092608
(87) International publication number: WO 2022/126999

(57) **Abstract**

The present disclosure discloses a surgical instrument (400), a slave operating equipment (200) using the surgical instrument (400) and a surgical robot having the slave operating equipment (200). The surgical instrument (400) includes a drive device (410) which includes a plurality of guide mechanisms (471, 472, 473) for guiding a plurality of cables (412, 422, 432, 442, 452). The cables (412, 422, 432, 442, 452) are in a divergent state before being guided, and converge to form a cable bundle after being guided by the plurality of guide mechanisms (471, 472, 473) in the drive device (410), and finally extend to wrist (430) and end effector (440) of the surgical instrument (400) from an edge of the drive device (410). In this way, the purity of cables (412, 422, 432, 442, 452) have more routing space 410 by means of the routing modes of the divergent state and the cable bundle state, and the cables (412, 422, 432, 442, 452) will not be interfered with each other.

## Description

### CROSS-REFERENCE TO RELATED APPLICATIONS

The present disclosure claims priority to Chinese patent application No. CN 202011512462.3, filed with the China National Intellectual Property Administration on December 19, 2020. All disclosures of the Chinese patent application are incorporated herein by reference.

### TECHNICAL FIELD

The subject matter herein generally relates to the field of medical instruments, in particular to a surgical instrument, a slave operating equipment using the surgical instrument, and a surgical robot having the slave operating equipment.

### BACKGROUND

Minimally invasive surgery refers to a surgical method of performing a procedure in a human body cavity using modern medical instruments such as laparoscope, thoracoscope, and so on. Compared with traditional surgery modes, minimally invasive surgery has advantages of being small in trauma, light in pain, fast in recovery, and the like.

With advances in science and technology, minimally invasive surgical technologies are increasingly mature and widely used. The minimally invasive surgical robot usually includes a master operation console and a slave operating equipment. The master operation console is used to send control commands to the slave operating equipment according to the operation of the doctor to control the slave operating equipment, and the slave operating equipment is used to respond to the control commands transmitted by the master operation console and perform corresponding surgical operations.

A surgical instrument that can be detached from the slave operating equipment is connected to the slave operating equipment. The surgical instrument includes a drive device and an end effector for performing surgery, and an elongate shaft for connecting the end effector and the drive device. The drive device connects the surgical instrument to the slave operating equipment and receives a driving force from the slave operating equipment to drive the end effector to move. The drive device is connected to the end effector through a cable, and the drive device manipulates the movement of the end effector through the cable. The drive device includes a plurality of drive units, the plurality of drive units are engaged with a plurality of actuators on the slave operating equipment, the proximal end of the cable is wound around the drive unit, the distal end of the cable is connected with the end effector, and the drive unit drives the end effector to move through the cable. The more the number of drive units and cables increases, the more degrees of freedom of motion the end effector has, that is, the more flexible the end effector is. However, due to the limited space in the drive device, the more cables there are, the more complicated the routing of the cables in the drive device will be. The complex routing not only makes the assembly process of the drive device difficult and the entire drive device structure complicates, and there is also a risk of mutual interference between cables.

### SUMMARY

Based on this, in order to solve the above problems, the present disclosure provides a surgical instrument, the surgical instrument includes a drive device, a plurality of cables, an elongate shaft and an end instrument located at a distal end of the elongate shaft, the plurality of cables are connected between the drive device and the end instrument, wherein, the drive device includes:
a plurality of drive units, the plurality of cables are connected, at an end, to the plurality of drive units;
a first guide mechanism and a second guide mechanism, the plurality of cables are guided by the first guide mechanism to form a cable bundle, the cable bundle includes a first segment cable bundle located between the first guide mechanism and the second guide mechanism, a second segment cable bundle located between the second guide mechanism and the proximal end of the elongate shaft, the first segment cable bundle extends to the second guide mechanism in a first direction, and the second segment cable bundle extends toward the proximal end of the proximal end of the elongate shaft in a second direction different from the first direction.

Preferably, the second guide mechanism is located at the proximal end or distal end of the first guide mechanism.

Preferably, the first segment cable bundle is substantially perpendicular to the cable bundle segments of the plurality of cables between the plurality of the drive units and the first guide mechanism.

Preferably, the proximal end of the elongate shaft is located at the edge of the drive device.

Preferably, the first direction and the second direction are non-parallel.

Preferably, the drive device further includes a third guide mechanism located near the proximal end of the elongate shaft, the second segment cable bundle is located between the second guide mechanism and the third guide mechanism, the cable bundle includes a third segment cable bundle located between the third guide mechanism and the proximal end of the elongate shaft, and the third segment cable bundle extends into the elongate shaft in a third direction opposite the first direction.

Preferably, the first guide mechanism includes a plurality of first pulleys, the first pulley includes a first axle and a first guide part for guiding one of the plurality of cables, the first guide part is rotatably mounted on the first axle, and the first axles of the plurality of first pulleys are substantially perpendicular to the rotational axes of the plurality of drive units.

Preferably, the second guide mechanism includes a plurality of second pulleys, the second pulley include a second axle and a second guide part for guiding one cable segment of the first segment cable bundle, the second guide part is rotatably mounted on the second axle, and the second axles of the plurality of second pulleys are substantially perpendicular to the rotational axes of the plurality of drive units.

Preferably, the third guide mechanism includes a plurality of third pulleys, the third pulley include a third axle and a third guide part for guiding one cable segment of the second segment cable bundle, the third guide part is rotatably mounted on the third axle, and the third axles of the plurality of third pulleys are substantially perpendicular to the rotational axes of the plurality of drive units.

Preferably, the plurality of cables includes a first cable, an acute angel is formed between the first axle of the pulley that guides the first cable among the plurality of first pulleys and the second axle of the pulley that guides the first cable among the plurality of second pulleys.

Preferably, the plurality of cables includes a second cable, the first axle of the pulley that guides the second cable among the plurality of first pulleys is substantially perpendicular to the second axle of the pulley that guides the second cable among the plurality of second pulleys.

Preferably, the plurality of cables further includes a third cable, and the first axle of the pulley that guides the third cable among the plurality of the first pulleys is substantially parallel to the second axle of the pulley that guides the third cable among the plurality of the second pulleys.

Preferably, the cable segment of the first cable between the plurality of drive units and the first guide mechanism and the cable segment of the first cable in the first segment cable bundle are on a first plane. The cable segment of the first cable in the first segment cable bundle and the cable segment of the first cable in the second segment cable bundle are on a second plane, wherein the first plane intersects the second plane.

Preferably, the cable segment of the second cable between the plurality of drive units and the first guide mechanism and the cable segment of the second cable in the first segment cable bundle are on a third plane; the cable segment of the first cable in the first segment cable bundle and the cable segment of the cable in the second segment cable bundle are on a fourth plane, wherein the third plane is substantially perpendicular to the fourth plane.

Preferably, the cable segment of the third cable between the plurality of drive units and the first guide mechanism and the cable segment of the third cable in the first segment cable bundle are on a fifth plane; the cable segment of the third cables in the first segment cable bundle and the cable segment of the third cable in the second segment cable bundle are on a sixth plane, the fifth plane is substantially parallel to the sixth plane.

Preferably, the plurality of cables includes a plurality of first cable segments formed between the plurality of drive units and the first guide mechanism, and a lengthwise direction of any cable segment of the plurality of first cable segments is substantially perpendicular to the lengthwise direction of the first segment cable bundle.

Preferably, the cable segments of the plurality of cables in the first segment cable bundle are substantially parallel to each other.

Preferably, the lengthwise direction of the second segment cable bundle is substantially perpendicular to the lengthwise direction of the first segment cable bundle.

Preferably, the cable segments of at least two of the plurality of cables in the second segment cable bundle are substantially parallel to each other.

Preferably, a lengthwise direction of the third segment cable bundle is substantially parallel to a lengthwise direction of the first segment cable bundle.

Preferably, the lengthwise direction of the third segment cable bundle is substantially parallel to a lengthwise direction of the second segment cable bundle.

Preferably, the cable segments of the plurality of cables in the third segment cable bundle are substantially parallel to each other.

Preferably, at least one of the plurality of first pulleys of the first guide mechanism is adjustable to lie on different levels within the drive device.

Preferably, at least one of the plurality of second pulleys of the second guide mechanism is adjustable to lie on different levels within the device.

Preferably, the plurality of drive units are located on the vertices of a polygon.

Preferably, the first guide mechanism is located in a middle area of the plurality of drive units.

Preferably, the first guide mechanism and/or the second guide mechanism include conduits for guiding the plurality of cables.

A slave operating equipment, the slave operating equipment includes a mechanical arm and the surgical instrument, the surgical instrument is mounted on the mechanical arm, and the mechanical arm is configured to control the movement of the surgical instrument.

A surgical robot includes a master operating equipment and the slave operating equipment, and the slave operating equipment performs corresponding operations according to the instructions of the master operating equipment.

The plurality of drive units in the drive device of the surgical instrument of the present disclosure can be flexibly arranged, after being guided by the guide mechanisms located at different spatial positions, the plurality of cables of the surgical instrument are extended in the drive device in the form of wire bundles, so that the routing of the cables is neat and takes up less space, and the assembly process of the drive device is simple, and at the same time, there is no risk of mutual interference between the cables.

### BRIEF DESCRIPTION OF THE DRAWINGS

FIG. 1 is a schematic structural diagram of a master operation console of a surgical robot according to an embodiment of the present disclosure.
FIG. 2 is a schematic structural diagram of a slave operating equipment of a surgical robot according to an embodiment of the present disclosure.
FIG. 3 is a schematic structural diagram of a surgical instrument according to an embodiment of the present disclosure.
FIG. 4 is a schematic diagram of a surgical instrument according to an embodiment of the present disclosure.
FIG. 5 is a top view of a drive device according to an embodiment of the present disclosure.
FIG. 6 is a schematic diagram of a state in which the elongate shafts of a plurality of surgical instruments are close together according to an embodiment of the present disclosure.
FIG. 7 is a schematic structural diagram of a surgical instrument according to another embodiment of the present disclosure.
FIG. 8 is a top view of the cable wiring of the drive device according to an embodiment of the present disclosure.
FIG. 9 is a top view of the cable wiring of the drive device according to an embodiment of the present disclosure.
FIG. 10 is a schematic structural diagram of a first guide mechanism and a second guide mechanism of a drive device according to an embodiment of the present disclosure.
FIG. 11 and FIG. 12 are schematic diagrams of cable wiring of the surgical instrument according to the embodiment shown in FIG. 3 of the present disclosure.
FIG. 13A is a top view of the cable wiring of the embodiment shown in FIG. 11.
FIG. 13B is a schematic diagram of the distribution of a plurality of contact points between the cable and the second guide mechanism according to the embodiment shown in FIG. 13A of the present disclosure.
FIG. 14A to FIG. 14D are schematic diagrams illustrating other distributions of a plurality of contact points between the cable and the second guide mechanism according to an embodiment of the present disclosure.
FIG. 15 is a side view of cable wiring according to an embodiment of the present disclosure.
Fig. 16 is a schematic diagram of the routing of the cable at the fourth guide mechanism of the embodiment shown in Fig. 15.
Fig. 17 is a perspective view of the fourth guide mechanism in the embodiment shown in Fig. 15.
FIG. 18 is a schematic view of the cable passing through the fourth guide mechanism in the embodiment shown in FIG. 15.
FIG. 19A- FIG. 19D are schematic diagrams of the fourth guide mechanism in some other embodiments of the present disclosure.
FIG. 20 is a schematic diagram of a state in which a first tensioning mechanism is connected to a cable according to an embodiment of the present disclosure.
FIG. 21 is a perspective view of a first tensioning mechanism according to an embodiment of the present disclosure.
FIG. 22 is an exploded view of the first tensioning mechanism of the embodiment shown in Figure 21.
FIG. 23 is a schematic diagram of a first tensioning mechanism according to another embodiment of the present disclosure.
FIG. 24A- FIG. 24C are schematic diagrams of a process of straightening a wrist by a first tensioning mechanism according to an embodiment of the present disclosure.
FIG. 25 is a schematic diagram of a second tensioning mechanism according to an embodiment of the present disclosure.
FIG. 26 is a schematic diagram of the installation of a base and a guide assembly of a drive device according to an embodiment of the present disclosure.
FIG. 27 is an exploded view of a guide assembly according to an embodiment of the present disclosure.
FIG. 28 is a bottom view of a first housing of the guide assembly of the embodiment shown in FIG. 26.
FIG. 29 is a bottom view of the base of the embodiment shown in FIG. 26.

### DETAILED DESCRIPTION

For ease of understanding of the present disclosure, the present disclosure will be described more fully hereinafter with reference to the associated drawings. Some embodiments of the present disclosure are set forth in the accompanying drawings. This application may, however, be embodied in many different forms and is not limited to the embodiments described herein. Rather, these embodiments are provided for the purpose of providing a more thorough and complete understanding of the disclosure of the present disclosure.

It should be noted that when an element is referred to as being "disposed on" another element, it may be directly on another element or intervening elements may also be present. When an element is considered to be "connected" to another element, it may be directly connected to another element or intervening elements may be present at the same time. When an element is considered to be "coupled" to another element, it may be directly coupled to another element or intervening elements may be present at the same time. As used herein, the terms "vertical", "horizontal", "left", "right" and similar expressions used herein are intended for purposes of illustration only, it should be understood that such terms do not denote absolute orientation. As used herein, the terms "distal end" and "proximal end" are common terms in the art of interventional medical devices, where "distal end" refers to the end far away from the operator during the surgical procedure, and the "proximal end" refers to the end close to the operator during the surgical procedure.

Unless otherwise defined, all technical and scientific terms used herein have the same meaning as commonly understood by one skilled in the art. The terminology used herein is for the purpose of describing particular embodiments only and is not intended to be limiting of the invention. As used herein, the term "and/or" includes all combinations of one or more of the associated listed items.

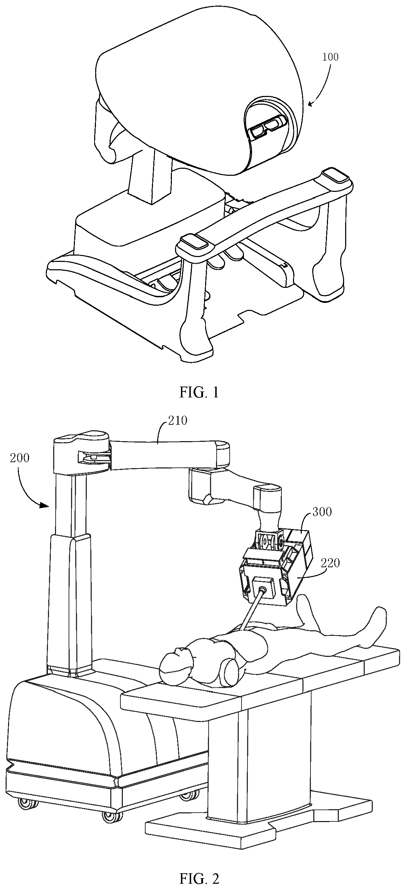

A minimally invasive surgical robot generally includes a slave operating equipment and a master operation console. FIG. 1 shows a master operation console 100 according to an embodiment of the present disclosure, and FIG. 2 shows a slave operating equipment 200 according to an embodiment of the invention. A surgeon performs operations of relevant control for the slave operating equipment 200 on the mater operation console 100, and the slave operating equipment 200 performs a surgical operation on the human body according to the input instructions from the mater operation console 100. The master operation console 100 and the slave operating equipment 200 can be set in one operating room or in different rooms, and even the mater operation console 100 and the slave operating equipment 200 can be far apart. For example, the master operation console 100 and the slave operating equipment 200 are located in different cities, and the master operation console 100 and the slave operating equipment 200 can transmit data in a wired manner or wirelessly. For example, the mater operation console 100 and the slave operation equipment 200 are located in the same operating room, and the two are in a wired manner for data transmission; another example is that the master operation console 100 and the slave operating equipment 200 are in different cities, and long-distance data transmission is performed between the two through 5G wireless signals.

The slave operating equipment 200 includes a robotic arm 210 and an actuating device 220 disposed at a distal end of the robotic arm 210. The surgical instrument 300 for performing a surgical operation is connected to the actuating device 220, and the actuating device 220 has a plurality of actuators for actuating surgical instruments to move. A plurality of surgical instruments 300 can be connected to one actuating device 220, and the distal ends of the plurality of surgical instruments 300 enter the human body through an incision, thereby reducing the number of the surgical incisions and making postoperative recovery faster.

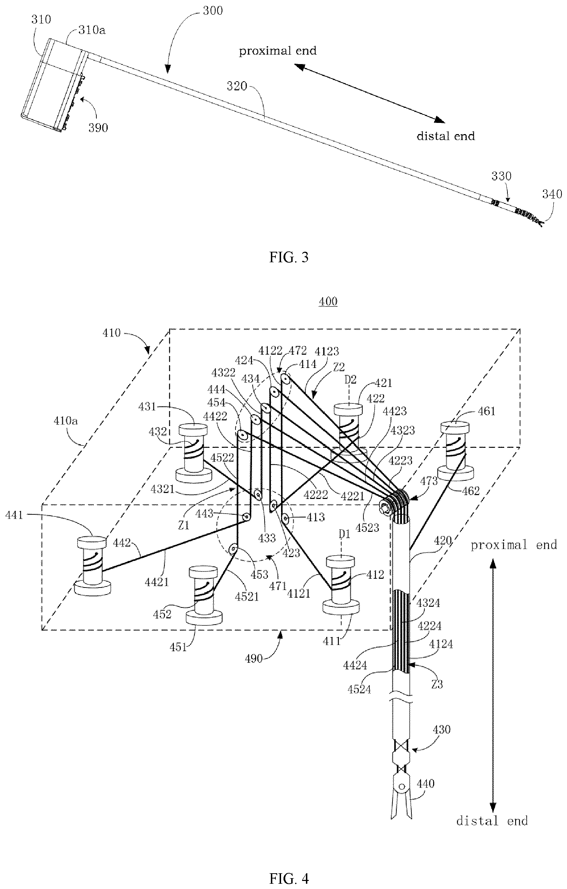

A surgical instrument according to an embodiment of the present disclosure is shown in FIG. 3. The surgical instrument 300 includes a drive device 310, an elongate shaft 320 and an end instrument located at the distal end of the elongate shaft 320. The end instrument includes a wrist 330 and/or an end effector 340, the drive device 310 is engaged with the actuating device 220 through an engaging portion 390, and a plurality of drive units inside the drive device 310 are connected to the wrist 330 and the end effector 340 by a plurality of cables, and the drive units drive the wrist 330 and/or the end effector 340 by operating the plurality of cables The cable can be flexible, and can also be a segmented strip including a flexible segment and a rigid segment strip, and the end effector 340 can be electric cautery, scissors, clamps, imaging device etc. In some other embodiments, the distal end of the elongate shaft is only connected to the wrist, and the movement of the wrist performs the action of pressing or lifting the tissue.

A surgical instrument according to an embodiment of the present disclosure is shown in FIG.4, the drive device 410 of the surgical instrument 400 has a plurality of drive units 411, 421,431, 441, 451 and 461, and the drive units 411, 421,431, 441, 451 and 461 are arranged in a polygon, each of the drive units 411, 421,431, 441, 451 and 461 is located on a vertex of the polygon. The number of the drive units in this embodiment is six, so that the plurality of drive units 411, 421,431, 441, 451,461 are respectively located on the vertices of a hexagon. In other embodiments, the number of the drive units may also be other number, such as five, seven, etc.

The proximal ends of the plurality of cables 412, 422, 432, 442, 452 are respectively wound around the plurality of drive units 411, 421,431, 441, 451, and the distal ends of the plurality of cables 412, 422, 432, 442, 452 are connected to the wrist 430 and the end effector 440 of the end instrument through the elongate shaft 420. The plurality of the drive units 411, 421,431, 441, 451rotate around their axes to pull or release the plurality of cables 412, 422, 432, 442, 452 to drive the wrist 430 and the end effector 440 to move.

The plurality of cables 412, 422, 432, 442, 452 extend into the elongate shaft 420 after being guided by a plurality of guide mechanisms inside the drive device 410. The plurality of guide mechanisms include a first guide mechanism 471, a second guide mechanism 472 and a third guide mechanism 473, the first guide mechanism 471 is located in the middle area of the polygon formed by the plurality of drive units 411, 421,431, 441, 451, the second guide mechanism 472 is located closer to proximal end of the drive device 410 than the first guide mechanism 471, the third guide mechanism 473 is located at the proximal end of the elongate shaft 420. One end of the cable 462 is wound around the drive unit 461, and the other end the cable 462 is wound around the proximal end of the elongate shaft 420. The drive unit 461 rotates around its rotation axis to pull or release the cable 462 to drive the elongate shaft 420 to rotate around its axis. In other embodiments, the second guide mechanism 472 may be located closer to the distal end of the drive device 410 than the first guide mechanism 471.

A plurality of cables 412, 422, 432, 442, 452 are guided by the first guide mechanism 471 and then converge into a cable bundle and extend to the end instrument in the form of the cable bundle. The cable bundle includes a first segment cable bundle Z1, a second segment cable bundle Z2 and a third segment cable bundle Z3. The first segment cable bundle Z1 extends along the first direction to the second guide mechanism 472 and is guided by the second guide mechanism 472 to form the second segment cable bundle Z2. The second segment cable bundle Z2 extends along a second direction non-parallel to the first direction to the third guide mechanism 473 and is guided by the third guide mechanism 473 to form a third segment cable bundle Z3, and the third segment cable bundle Z3 extends through the elongate shaft 420 to the wrist 430 and the end effector 440.

The first guide mechanism 471 is located in the middle area of the polygon formed by the plurality of drive units 411, 421,431, 441, 451, and the plurality of cables 412, 422, 432, 442, 452 extend to the first guide mechanism 471 in a manner of converging from the vertices of the polygon to the middle area of the polygon, and then converge to the first segment cable bundle Z1 after being guided by the first guide mechanism 471. Specifically, the plurality of cables 412, 422, 432, 442, 452 form a plurality of the first cable segments 4121, 4221, 4321, 4421, 4521 between the plurality of drive units 411, 421,431, 441, 451 and the first guide mechanism 471, the plurality of first cable segments 4121, 4221, 4321, 4421, 4521 are projected onto a projection plane S1which is perpendicular to the rotational axis of any one of the plurality of drive units 411, 421,431, 441, 451, the projection line segments do not intersect.

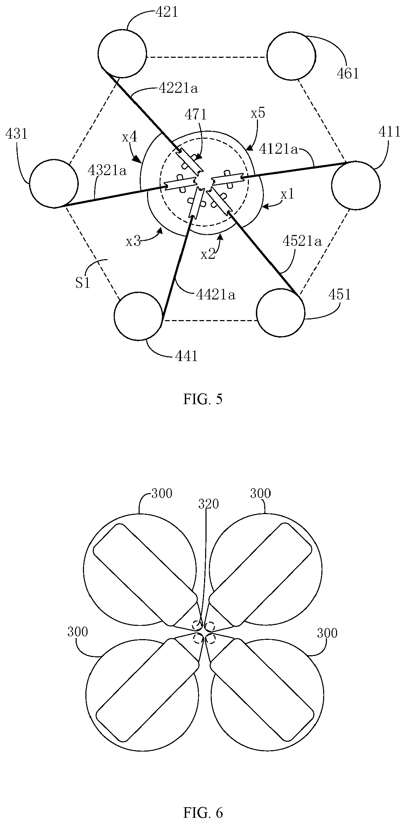

As shown in FIG.5, the plurality of first cable segments 4121, 4221, 4321, 4421, 4521 forms the projection line segments 4121a, 4221a, 4321a, 4421a, 4521a by projecting on the projection plane S1, and the projection line segments 4121a, 4221a, 4321a, 4421a, 4521a do not intersect. There is a plurality of first angles x1, x2, x3, x4, x5 between the plurality of first cable segments 4121, 4221, 4321, 4421, 4521, and the plurality of first angles x1, x2, x3, x4, x5 take the center point of the first guide mechanism 471 as the vertex, and the edges of the plurality of first angles x1, x2, x3, x4, x5 are along the projection line segments 4121a, 4221a, 4321a, 4421a, 4521a. The plurality of first angles x1, x2, x3, x4, x5 refer to the smaller angle formed by two adjacent projection line segments among the projection line segments 4121a, 4221a, 4321a, 4421a, 4521a, that is, the first angles x1, x2, x3, x4, x5 are all less than or equal to 180°. Since the first guide mechanism 471 is located in the middle area of the plurality of drive units 411, 421, 431, 441 and 451, the sum of the plurality of first angles x1, x2, x3, x4, x5 equals 360°, so that each of the plurality of first angles x1, x2, x3, x4, x5 is as large as possible, thus there is more space for wiring between the plurality of cables 412, 422, 432, 442, 452, avoiding mutual interference between the plurality of cables 412, 422, 432, 442, 452.

Referring again to FIG. 4, the first segment cable bundle Z1 is located between the first guide mechanism 471 and the second guide mechanism 472, and the first segment cable bundle Z1 includes a plurality of second cable segments 4122, 4222, 4322, 4422, 4522, with substantially the same length direction, of the plurality of cables 412, 422, 432, 442,452. The second cable segments 4122, 4222, 4322, 4422, 4522 are substantially parallel to each other and are closely spaced, so that the space occupied by the second segment cable bundle Z1 is as small as possible, so as to make full use of the space inside the drive device 410 to arrange other mechanisms, such as a cable tensioning mechanism. In other embodiment, the plurality of second cable segments of the second segment cable bundle Z1 may not all be parallel, but the directions of the plurality of second cable segments need to be substantially consistent.

The plurality of first cable segments 4121, 4221, 4321, 4421, 4521 are in a divergent state from the first guide mechanism 471 to the plurality of drive units 411, 421, 431, 441, 451, so that the plurality of first cable segments 4121, 4221, 4321, 4421, 4521 occupy the first space in the horizontal direction at the distal end of the drive device 410. The direction of the first segment cable bundle Z1 is perpendicular to the direction of any one of the plurality of first cable segments 4121, 4221, 4321, 4421, 4521, and the second guide mechanism 472 is located at the proximal end of the first guide mechanism 471, therefore, the first segment cable bundle Z1 between the first guide mechanism 471 and the second guide mechanism 472 occupies the second space in a vertical direction at the middle area of the drive device 410. The first space is substantially perpendicular to the second space, and the first space and the second space only intersect at the first guide mechanism 471, but the first space and the second space do not overlap, so that the first segment cable bundle Z1 will not affect the routing of the plurality of first cable segments 4121, 4221, 4321, 4421, 4521, nor will it interfere with the plurality of the first cable segments 4121, 4221, 4321, 4421, 4521. In some other embodiments, the first segment cable bundle Z1 may not be perpendicular to the plurality of first cable segments 4121, 4221, 4321, 4421, 4521, or the cable segments of the first segment cable bundle Z1 are perpendicular to the plurality of first cable segments 4121, 4221, 4321, 4421, 4521, so that the second space where the first segment cable bundle Z1 is located has a certain included angle with the first space where the plurality of first cable segments 4121, 4221, 4321, 4421 and 4521 are located. However, since the two spaces do not overlap, the first segment cable bundle Z1 will still not affect the routing of the plurality first cable segments 4121, 4221, 4321, 4421, 4521, nor will it interfere with the plurality first cable segments 4121, 4221, 4321, 4421, 4521.

The second segment cable bundle Z2 is located between the second guide mechanism 472 and the third guide mechanism 473, the second segment cable bundle Z2 includes a plurality of third cable segments 4123, 4223, 4323, 4423, 4523 of the cables 412,422, 432, 442, 452, and the plurality of third cable segments 4123, 4223, 4323, 4423, 4523 have approximately same direction and close to each other, so that the third space occupied by the second segment cable bundle Z2 is also as little as possible. In some other embodiments, at least two or more cable segments are parallel to each other among the plurality of third cable segments of the second segment cable bundle Z2, so that the third space occupied by the second segment cable bundle Z2 is less.

The second segment cable bundle Z2 extends toward the position where the proximal end of the elongate shaft 420 is located, and the direction (first direction) of the first segment cable bundle Z1 is substantially perpendicular to the direction (second direction) of the second segment cable bundle Z2. The second guide mechanism 472 isolates the second segment cable bundle Z2 and the plurality of first cable segments 4121, 4221, 4321, 4421, 4521 at different horizontal planes in the drive device 410 respectively, that is, the second segment cable bundle Z2 is located at a proximal end of the drive device 410, and the plurality of first cable segments 4121, 4221, 4321, 4421, 4521 are located at a distal end of the drive device 410, so that the third space where the second segment cable bundle Z2 is located will not overlap with the first space where the first cable segments 4121, 4221, 4321, 4421, 4521 are located. In this way, the second segment cable bundle Z2 will not affect the routing of the plurality of first cable segments 4121, 4221, 4321, 4421, 4521, nor will interfere with the first cable segments 4121, 4221, 4321, 4421,4521, and the third space where the second segment cable bundle Z2 is located will not overlap with the second space where the first segment cable bundle Z1 is located, so that the second segment cable bundle Z2 also will not affect the routing of the first segment cable bundle Z1. In other embodiments, the second segment cable bundle Z2 may not be perpendicular to the first segment cable bundle Z1, as the third guide mechanism 473 is not located in the first space where the plurality of first cable segments 4121, 4221, 4321, 4421, 4521 are located, so that even if the second segment cable bundle Z2 is not perpendicular to the first segment cable bundle Z1, the third space where the second segment cable bundle Z2 is located will still not overlap with the first space where the first cable segments 4121,4221, 4321, 4421, 4521 are located. The second segment cable bundle Z2 will not affect the routing of plurality of first cable segments 4121, 4221, 4321, 4421, 4521, and the third space where the second cable segment Z2 is located will not overlap with the second space where the second space where the first cable segment Z1 is located, so that the second cable segment Z2 will not affect the routing of the first segment cable bundle Z 1.

The first guide mechanism 471 includes a plurality of first pulleys 413, 423, 433, 443, 453, the second guide mechanism 472 includes a plurality of second pulleys 414, 424, 234, 444, 454. Each of the plurality of first pulleys 413, 423, 433, 443, 453 and the plurality of second pulleys 414, 424, 234, 444, 454 includes an axle and a guide part for guiding the plurality of cables 412,422,432, 442, 452, the guide part is rotatably mounted on the axle. In other embodiments, the guide part and the axle can also be fixedly connected, so that the guide part and the axle rotate together.

The axle of the plurality of first pulleys 413, 423, 433, 443, 453 is substantially perpendicular to the rotation axis of any one of the plurality of drive units 411, 421, 431, 441, 451, and the length direction of the first segment cable bundle Z1 is the same or approximately the same as the rotation axis of the plurality of drive units 411, 421, 431, 441, 451. Adjusting the height of the first guide mechanism 471 and the tensioning mechanism (not shown) in the drive device 410, that is, adjusting the position of the first guide mechanism 471 in the drive device 410 along the direction of a proximal end or a distal end of the drive device 410, will not affect the routing of the plurality of first cable segments 4121, 4221, 4321, 4421, 4521 and the orientation of the first segment cable bundle Z1, and will not affect the transmission efficiency of the first segment cable bundle Z1. Therefore, the height of the first guide mechanism 471 can be adjusted according to the different cable routing requirements or the layout requirements of various components of the drive device 410.

Taking the routing of the first cable 412 as an example, the first cable 412 is guided by the first pulley 413 of the first guide mechanism 471 and then extends to the second guide mechanism 472, the axle of the first pulley 413 is perpendicular to the rotation axis D1 of the drive unit 411. The first cable 412 enters a groove along a tangential direction of the groove which is located on the guide part of the pulley 413 and configured to accommodate the first cable 412, in this way, no matter how to adjust the height of the first pulley 413 in the drive device 410, the first cable 412 can enter or leave the groove along the tangent of the groove of the first pulley 413, so that adjusting the height of the first pulley 413 will not affect the first cable 412 entering the groove of the first pulley 413, and will not affect transmission efficiency of the first cable 412, in this way, the height of the first pulley 413 can be adjusted, that is, the height of the first guide mechanism 471 can be adjusted.

The axles of the plurality of second pulleys 414, 424, 234, 444, 454 are also substantially perpendicular to the rotation axis of any one of the plurality of drive units 411, 421, 431, 441, 451, so that adjusting the height of the second guide mechanism 472 and/or the height of the tensioning mechanism, will not affect the transmission efficiency of the first segment cable bundle Z1 and the second segment cable bundle Z3. Taking the first cable 412 as an example, the first cable 412 extends to the third guide mechanism 473 after being guided by the second pulley 414 of the second guide mechanism 472, as the axle of the second pulley 413 is perpendicular to the rotation axis D1 of the drive unit 411, no matter how the height of the pulley 414 in the drive device 410 is adjusted, the second cable segment 4122 and the third cable segment 4123 of the first cable 412 can enter or leave the groove on the guide part of the second pulley 412 along the tangential direction of the groove, so that the height of the second guide mechanism 472 in the drive device 410 can be adjusted, as well as the first guide mechanism 471.

The proximal end of the elongate shaft 420 is located at an edge of the housing 410a of the drive device 410 (the edge refers to a position near the periphery of the housing, i.e., near the intersection of two or three sides of the housing), which can make the elongate shafts 420 of the plurality of surgical instruments be close together, and so that the distal ends of the plurality of elongate shaft 420 can be brought together to insert into the human body through an incision, thereby reducing the number of surgical incisions. FIG. 6 is a top view of a plurality of surgical instruments 300, the elongate shafts 320 of the surgical instruments 300 are located at edges of the surgical instruments 300, therefore, the elongate shafts 320 of the plurality of surgical instruments 300 can be brought together, and thus the distal end instruments of the elongate shafts 320 are inserted into the human body through an incision.

Referring again to FIG. 4, the third segment cable bundle Z3 includes a plurality of third cable segments 4124, 4224, 4324, 4424, 4524 of a plurality of cables 412, 422, 432, 442, 452, the third cable segments 4124, 4224, 4324, 4424, 4524 are substantially parallel to each other, the third guide mechanism 473 is located at the edge of the surgical instrument 400, and the third guide mechanism 473 is located near the proximal end of the elongate shaft 420.

The third guide mechanism 473 includes a plurality of third pulleys for guiding the plurality of cables 412, 422, 432, 442, 452. Each of the pulleys of the third pulleys of the third guide mechanism 473 is close to each other, so that each of the cable segments of the third cable segments 4124, 4224, 4324, 4424, 4524 of the third segment cable bundle Z3 can be brought closer to each other, and the third segment cable bundle Z3 can extend through the elongate shaft 420 with less space to reach the wrist 430 and the end effector 440.

In one embodiment, the axles of the plurality of third pulleys are arranged similarly to the axles of the plurality of the first pulleys and the axles of the plurality of the second pulleys, and are also perpendicular to the rotation axis of any one of the plurality of drive units 411, 421, 431, 441, 451, therefore, the height of the third guide mechanism 473 in the drive device 410 can be adjusted like the first guide mechanism 471 and the second guide mechanism 472.

The heights of the first guide mechanism 471, the second guide mechanism 472 and the third guide mechanism 473 in the drive device 410 are adjustable, not only the first guide mechanism 471 or the second guide mechanism 472 or the third guide mechanism 473 as a whole can be adjusted, but also a height of an element of the first guide mechanism 471 or the second guide mechanism 472 or the third guide mechanism 473 in the drive device 410 can be adjusted. For example, the height of the first guide mechanism 471 as a whole is adjustable in the drive device 410, alternatively, the height of one or more pulleys of the plurality of the first pulleys 413, 423, 433, 443 of the first guide mechanism 471 in the drive device 410 can be adjusted.

By adjusting the height of the first guide mechanism 471 and/or the second guide mechanism 472, and/or the third guide mechanism 473 in the drive device 410, the tension of the plurality of cables 412, 422, 432, 442, 452 and the wiring of the plurality of cables 412, 422, 432, 442, 452 at a horizontal height in the drive device 410 can be adjusted.

The first cable 412 is guided by the first pulley 413 of the first guide mechanism 471 and then guided by the second pulley 414 of the second guide mechanism 472 to extend to the third guide mechanism 473. The first cable 412 has a first cable segment 4121 located between the drive unit 411 and the first pulley 413, a second cable segment 4122 located between the first pulley 413 and the second pulley 423, and a third cable segment 4223 located between the second pulley 423 and the third guide mechanism 473. The first cable segment 4121 and the second cable segment 4122 are located on a first plane, the second cable segment 4122 and the third cable segment 4123 are located on a second plane. The included angle between the axle of the first pulley 413 and the axle of the second pulley 414 is an acute angle, that is, the axle of the pulley 413 and the axle of the pulley 414 are non-perpendicular and non-parallel to the first plane, so that the first plane and the second plane intersect but do not overlap.

In one embodiment of the present disclosure, the first cable segment 4221 of the second cable 422 and the second cable segment 4222 of the second cable 422, among the plurality of cables 412, 422, 432, 442, 452, are located on a third plane, and the second cable segment 4222 and the third cable segment 4223 of the second cable 422 are located on a fourth plane. The axis of the first pulley 423 for guiding the second cable 422 is substantially perpendicular to the axis of the second cable 424 for guiding the second cable 422, so that the third plane is substantially perpendicular to the fourth plane.

In one embodiment of the present disclosure, the first cable segment 4321 of the third cable 432 and the second cable segment 4322 of the third cable 422, among the plurality of cables 412, 422, 432, 442, 452, are located on a fifth plane, and the second cable segment 4322 and the third cable segment 4323 of the second cable 432 are located on a sixth plane. The axle of the first pulley 433 for guiding the third cable 432 is substantially parallel to the axle of the second pulley 434 for guiding the third cable 432, so that the fifth plane is substantially parallel to the sixth plane.

It can be understood that, the plurality of cables 412, 422, 432, 442, 452 in the drive device 400 may all be routed in the same way as the first cable 412; or some of the plurality of cables 412, 422, 432, 442, 452 are routed in the same way as the first cable 412, while others are routed in the same way as the second cable 422 or the third cable 432; or some of the plurality of cables 412, 422, 432, 442, 452 are routed in the same way as the second cable 422, while others are routed in the same way as the third cable 432.

After the plurality of cables 412, 422, 432, 442, 452 in the drive device 400 are routed in the routing manner described above, at least one of the plurality of cables 412, 422, 432, 442, 452 changes plane after being guided by the second guide mechanism 472, that is, the plane where the cable segment, at two sides of the first guide mechanism 471, of the least one cable of the cables 412, 422, 432, 442, 452 is located intersect but do not overlap with plane where the cable segment, at two sides of the second guide mechanism 472, of the cable is located. Therefore, the second segment cable bundle Z2 formed by the plurality of cables 412, 422, 432, 442, 452 after being guided by the second guide mechanism 472 extends to the edge of the drive device 410, and the plurality of cables 412, 422, 432, 442, 452, after being guided by the second guide mechanism 472, can enter the elongate shaft 420 from the edge of the drive device 472, in this way, the elongate shaft 472 can be mounted to the edge of the drive device 410.

In the embodiment shown in FIG. 4, the second segment cable bundle Z2, after being guided by the third guide mechanism 473, enters the elongate shaft 420 located at the edge of the drive device 410, an axis of the elongate shaft 420 is perpendicular to an engaging portion 490, such that a proximal end of the actuating device 220 is thereby engaged with the engaging portion 490 of the drive device 410. In other embodiment, the engaging portion 490 of the drive device 410 can be located at a proximal end of the drive device 470, such that a distal end of the actuating device 220 is engaged with the engaging portion 490 of the drive device 140.

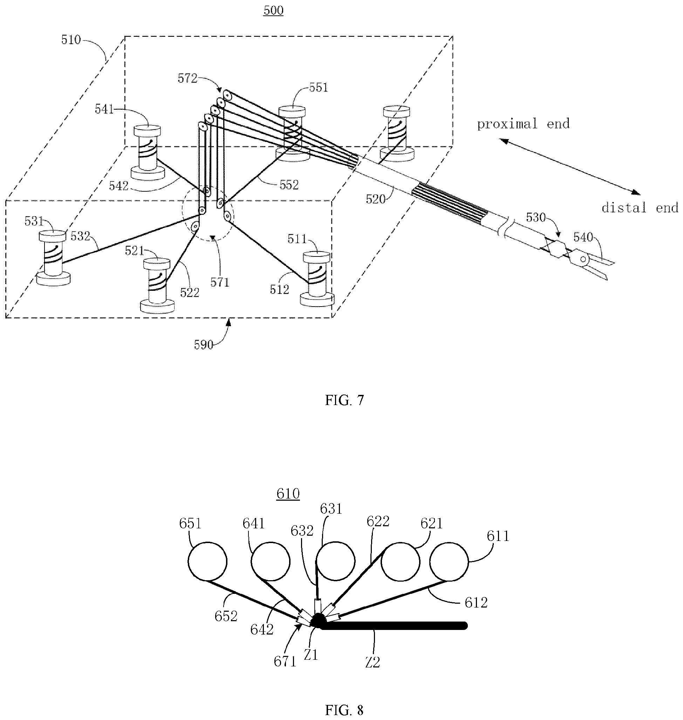

A surgical instrument according to an embodiment of the present disclosure is shown in FIG. 7, proximal ends of the plurality of cables 512, 522, 532, 542, 552 are fixed to a plurality of drive units 511, 521, 531, 541, 551, and distal ends of the plurality of cables 512, 522, 532, 542, 552 are guided by the first guide mechanism 571 and the second guide mechanism 572 and extend to the edge of the drive device 410 in the form of a cable bundle, and then directly enter into the elongate shaft 520, and finally extend to the wrist 530 and the end effector 540. Compared with the embodiment shown in FIG. 4 that uses three guide mechanisms to guide the plurality of cables, in the present embodiment, only two guide mechanisms are used, the friction between the cables and the guide mechanisms can be reduced by setting fewer the guide mechanisms, the driving force consumption of the cables is less, and the transmission efficiency of the cables for the driving force is higher. However, since the engaging portion 590 of the drive device 510 is parallel to the axis of the elongate shaft 520, the side surface of the actuating device 220 is engaged with the engaging portion 590 of the drive device 510, which makes the process of installing the surgical instrument 510 on the actuating device 220 inconvenient.

A drive device of the surgical instrument according to an embodiment of the present disclosure is shown in FIG. 8. FIG. 8 is a top view of the drive device 610. A plurality of drive units 611, 621, 631, 641, 651 of the drive device 610 are arranged in a row, and the first guide mechanism 671 is located at a side of the plurality of the drive units 611, 621, 631, 641, 651. a plurality of cables 612, 622, 632, 642,652 drawn out from the plurality of drive units 611, 621, 631, 641, 651 converge to form a first segment cable bundle Z1 after being guided by a first guide mechanism 671, and the first segment cable bundle Z1 converges to forms a second segment cable bundle Z2 after being guided by a second guide mechanism (not shown). The second segment cable bundle Z2 extends to the edge of the drive device 610 and then enters directly into the elongate shaft, or enters into the elongate shaft after being guided by a third guide mechanism. Although the first guide mechanism 671 in this embodiment is not located in the middle area of the plurality of drive units 611, 621, 631, 641, 651, and the angles between the cable segments of the plurality of cables 612, 622, 632, 642, 652 between the plurality of drive units 611, 621, 631, 641, 651 and the first guide mechanism 671 are not greater than the angles shown in FIG. 5, but still greater than the angles in the prior art.

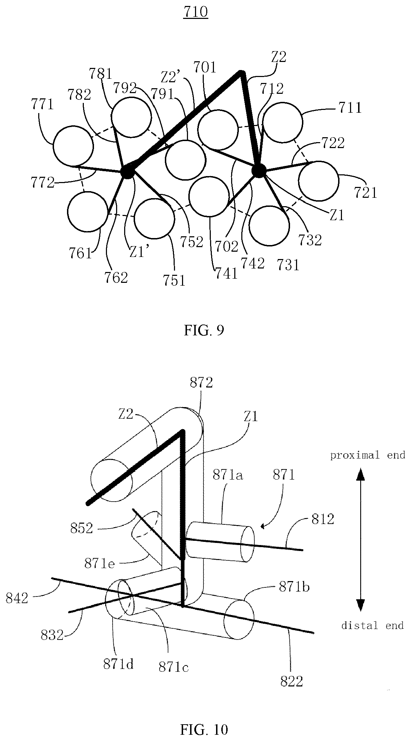

A drive device of the surgical instrument according to an embodiment of present disclosure is shown in FIG. 9, and FIG. 9 is a top view of the drive device 710. The drive device 710 includes a plurality of drive units 701, 711, 721, 731, 741, 751,761, 771, 781, 791, and in a polygon enclosed by the drive units 701, 711, 721, 731, 741, 751,761, 771, 781, 791, there are two first guide mechanisms and two second guide mechanisms (not shown), a plurality of cables 702, 712, 722,732, 742 drawn out from the drive units 701, 711, 721, 731, 741 converge to form a first segment cable bundle Z1 after being guided by a first guide mechanism, and the first segment cable bundle Z1 converges to form a second segment cable bundle Z2 after being guided by a second guide mechanism, a plurality of cables 752, 762, 772, 782, 792 drawn out from the drive units 751, 761, 771, 781, 791 converge to form a first segment cable bundle Z1' after being guided by another first guide mechanism, and the first segment cable bundle Z1' converges to form a second segment cable bundle Z2' after being guided by another second guide mechanism. The second segment cable bundles Z2 and Z2' extends to an edge of the drive device 710 and then enters directly into the elongate shaft of the surgical instrument, or enters into the elongate shaft of the surgical instrument after being guided by a third guide mechanism. Since the drive device 710 has more the drive units, the wrist and end effector of the surgical instrument have more degrees of freedom of motion.

A guide mechanism of the drive device according to embodiment of present disclosure is shown in FIG. 10, a first guide mechanism 871 and a second guide mechanism 872 are both in the form of pipes, and the first guide mechanism 871 includes a plurality of guiding conduits 871a,871b, 871c, 871d, 871e arranged in a dispersed state. The cables 812, 822, 832, 842, 852 converge to form a first segment cable bundle Z1 after being guided by the plurality of guiding conduits 871a,871b, 871c, 871d, 871e, and the first segment cable bundle Z1 forms a second segment cable bundle Z2 after being guided by the second guide mechanism 3872. The plurality of guiding conduits 871a, 871b, 871c, 871d, 871e of the first guide mechanism 871 can be arranged in different heights, so as to receive the cables of the drive units at different heights. The guide mechanism with the guiding conduits does not need complicated routing of the pulleys for guiding the cables, thus the structure is more simple. In other embodiments, the first guide mechanism can be a pipe-type structure shown as FIG. 10, and the second guide mechanism can be a multi-pulley-type structure shown as FIG. 4, or alternatively, the first guide mechanism can be a multi pulley-type structure shown as FIG. 4, and the second guide mechanism can be a pipe-type structure shown as FIG. 10.

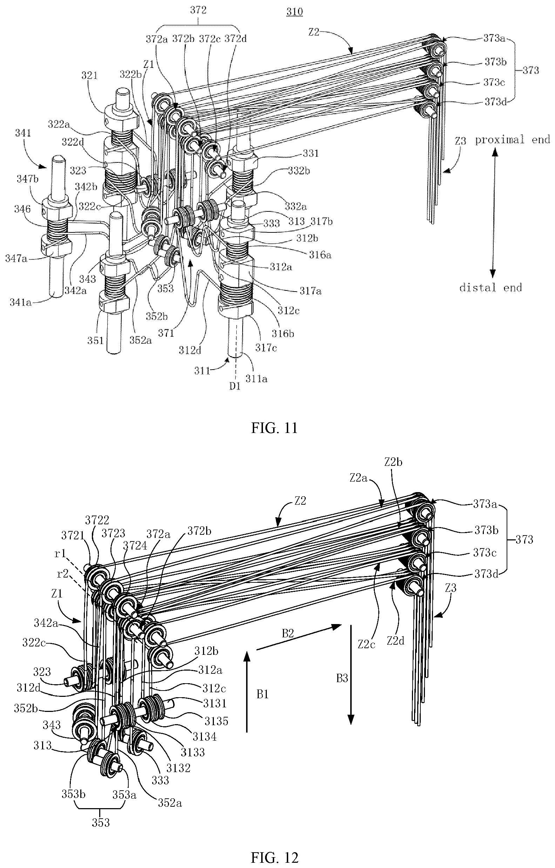

The internal structure of the drive device 310 of the surgical instrument 300 in the embodiment shown in FIG. 3 is shown in FIG. 11, the drive device 310 includes a plurality of drive units, a first guide mechanism 371, a second guide mechanism 372 and a third guide mechanism 373, and the drive units include a plurality of first drive units 311, 321 and a plurality of second drive units 331,341, 351. The first guide mechanism 371 is located a central area of the plurality of drive units, the second guide mechanism 372 is located at a proximal end of the first guide mechanism 371, and the third guide mechanism 373 is located at an edge of the housing 310a.

One end of each of the cables 312a, 312b, 312c, 312d, 322a, 322b, 322c, 322d is wound around the first drive units 311, 321, and one end of each of the cables 332a, 332b, 342a, 342b, 351a, 351b is wound around the second drive units 331, 341, 351. The cables 312a, 312b,312c, 312d, 322a, 322b, 322c, 322d, 332a, 332b, 342a, 342b, 351a, 351b converge to form a cable bundle after being guided by the first guide mechanism 371, and the cable bundle has multiple layers of sub-cable bundles, and the layers of the sub-cable bundles are substantially parallel to each other.

The cable bundles includes a first segment cable bundle Z1, a second segment cable bundle Z2 and a third segment cable bundle Z3, wherein the cables in the first segment cable bundle Z1 are substantially parallel. The first segment cable bundle Z1 extends to the second guide mechanism 372 along a direction (B1 direction shown in FIG. 12) and then converge to form a second segment cable bundle Z2 after being guided by the second guide mechanism 372, and at least two cables of the second segment cable bundle Z2 are parallel to each other. The second segment cable bundle Z2 extends to a third guide mechanism 373 along a second direction (B2 direction shown in FIG. 12), and converge to form a third segment cable bundle Z3 at a third direction (B3 direction shown in FIG. 12) after being guided by the third guide mechanism 373. The cables in the third segment cable bundle Z3 are substantially parallel to each other, and the third segment cable bundle Z3 extends through the elongate shaft 320 and is connected to the wrist 330 and the end effector 340. In other embodiment, the drive device 310 has only a first guide mechanism 371 and a second guide mechanism 372, thus similar to the embodiment shown in FIG. 7, the second segment cable bundle Z2 extends directly into the elongate shaft 320.

The first drive unit 311 includes a main shaft 311a, a first capstan 316a, a second capstan 316b, a first fixing block 317a, a second fixing block 317b and a third fixing block 317c, which are integrally formed, the second fixing block 317b is arranged at a proximal end of the main shaft 311a, and the third fixing block 317c is arranged at a distal end of the main shaft 311a. The diameters of the first capstan 316a and the second capstan 316b are different. The first capstan 316a is arranged between the first fixing block 317a and the second fixing block 317b, and the second capstan 316b is arranged between the second fixing block 317b and the third fixing block 317c. The first drive unit 311 is integrally formed, so that the first drive unit 311 is easy to manufacture and assemble. In other embodiments, the first drive unit may not be integrally formed, that is, the first capstan 316a and the second capstan 316b are independently installed on the main shaft, and can rotate relative to the main shaft respectively.

The first end of the cable 312a is fixed on the first fixing block 317a, and the cable 312a is wound on the first capstan 316a in a first winding manner (eg. counterclockwise). The first end of the cable 312b is fixed on the second fixing block 317b, and the cable 312b is wound on the first capstan 316a in a second winding manner (eg. clockwise) which is opposite to the first winding manner. The first ends of the cables 312c and 312d are respectively fixed on the third fixing block 317c, and are wound on the second capstan 316b in the first winding manner and the second winding manner respectively. The cables 312a, 312b, 312c and 312d extend from the first drive unit 311 to the first pulley group 313 of the first guide mechanism 371 in a manner of substantially parallel to each other, and further extend to the second guide mechanism 372 in such a substantially parallel manner after being guided by the first pulley group 313.

When the main shaft 311a of the first drive unit 311 rotates around the rotation axis D1, the first drive unit 311 can drive two joints on the wrist 330 with different distances from the end effector 340 to move by manipulating the cables 312a, 312b, 312c, 312d. The first drive unit 321 and the first drive unit 311 have substantially the same structure. The cables 322a, 322b, 322c, 322d extend from the first drive unit 311 to the first pulley 323 of the first guide mechanism 370 in a manner of substantially parallel with each other, and further extend to the second guide mechanism 372 in the substantially parallel manner after being guided by the first pulley 323.

The second drive unit 341 is also integrally formed. The second drive unit 341 includes a first fixing block 347a and a second fixing block 347b respectively disposed at the distal end and the proximal end of the main shaft 341a. A first capstan 346 is arranged between the first fixing block 347a and the second fixing blocks 347b, one end of the cable 342a is fixed on the first fixing block 347a, one end of the cable 342b is fixed on the second fixing block 347b, the cables 342a, 342b extend from the second drive unit 341 to the first pulley group 343 of the first guide mechanism 371 in a substantially parallel manner, and after being guided by the first pulley group 343, it still extends to the second guide mechanism 372 in a substantially parallel manner. The unit 341 drives the joints of the wrist 330 or the end effector 340 to move by manipulating the cables 342a, 342b. The structures of the second drive units 331, 351 and the second drive unit 341 are substantially the same, and the cables 332a and 332b extend from the second drive unit 331 through the first pulley 333 to the second guide mechanism 372 in a manner of substantially parallel with each other. The cables 352a and 352b extend from the second drive unit 351 to the second guide mechanism 372 through the first pulley 333 in a manner of substantially parallel with each other. In some other embodiments, the plurality of drive units of the drive device may all be the first drive unit or all be the second drive units.

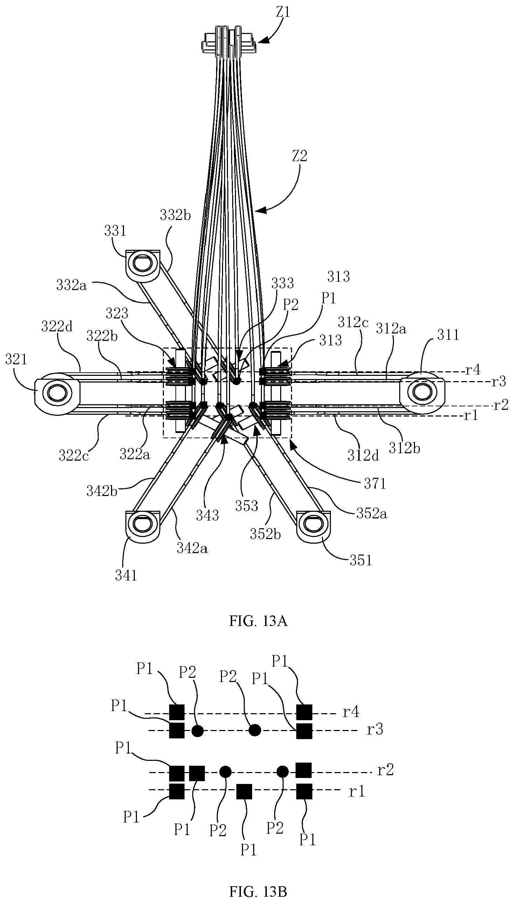

Similar to embodiment shown as FIG. 4, the sum of the plurality of first included angles between every two adjacent cables of the cables 312a, 312b, 312c, 312d, 322a, 322b, 322c, 322d, 332a, 332b, 342a, 342b, 351a, 351b drawn from different drive units is 360°, so that the plurality of cables 312a, 312b, 312c, 312d, 322a, 322b, 322c, 322d, 332a, 332b, 342a, 342b, 351a, 351b get more routing space. FIG. 13 is a top view of FIG. 11, with the second guide mechanism 372 hidden to more clearly show the routing of the cables. As shown in FIG. 13, the cable segments between the drive unit and the first guide mechanism 371 of the plurality of cables drawn from the same drive unit are substantially parallel to each other, so that the plurality of cables 312a, 312b, 312c, 312d , 322a, 322b, 322c, 322d, 332a, 332b, 342a, 342b, 351a, 351b will not interfere with each other in the first space between the drive units 311, 321, 331, 341, 351 and the first guide mechanism 371.

The plurality of first pulley groups 313, 323, 333, 343, and 353 of the first guide mechanism 371 are located on three different horizontal planes. In particular, two adjacent first pulleys of the plurality of first pulley groups 313, 323, 333, 343, and 353 are located on different horizontal planes, so that the entire first guide mechanism 371 occupies a smaller space volume in the horizontal direction on the premise that the cables in the first segment cable bundle Z1 do not interfere with each other. Specifically, the first pulley group 313 and the first pulley group 323 are located on the same horizontal plane at the proximal end of the drive device 310, the first pulley group 333 and the first pulley group 353 are located on the same horizontal plane at the distal end of the drive device 310, and the first pulley group 343 is located on the horizontal plane between the first pulley group 313 and the first pulley group 333.

The second guide mechanism 372 includes a plurality of second pulley group 372a, 372b, 372c, 372d for guiding the first segment cable bundle Z1, and each pulley in each of the plurality of second pulley groups 372a, 372b, 372c, 372d shares one axle, the axles of the plurality of second pulley groups 372a, 372b, 372c, 372d are substantially parallel to each other, and the plurality of second pulley groups 372a, 372b, 372c, 372d are respectively located on a plurality of first planes C1, C2, C3, On C4, each of the second pulley group can guide the plurality of cables, and one of the second pulley group can guide multiple cables from the same drive unit or multiple cables from different drive units.

As shown in FIG. 12, the second pulley group 372a of the second guide mechanism 372 includes an axle 3721 and a plurality of guide parts 3722, 3723, 3724 provided on the axle 3721. The guide part 3722 , the guide part 3723 and the guide part 3724 are respectively configured to guide the cable 322c from the second drive unit 321, the cable 342a from the first drive unit 341 and the cable 312d from the second drive unit 311.

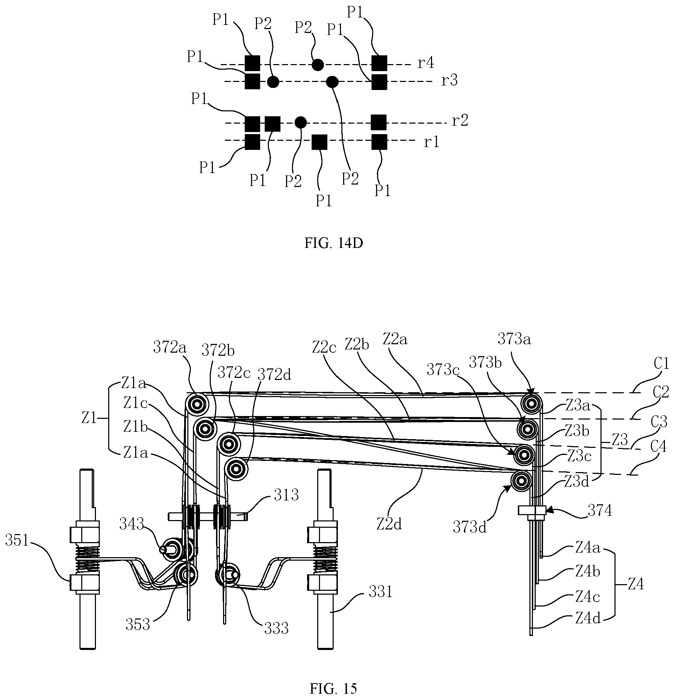

As shown in FIG. 15, the plurality of third pulley groups 373a, 373b, 373c, and 373d of the third guide mechanism 373 are also respectively located on the plurality of first planes C1, C2, C3, C4, so that the third pulley groups 373a, 373b, 373c, 373d and the plurality of second pulley groups 372a, 372b, 372c, 372d are arranged in pairs on the plurality of first planes C1, C2, C3, C4. That is, the second pulley group 372a and the third pulley group 373a are substantially located on the first plane C1, the second pulley group 372b and the third pulley group 373b are substantially located on the first plane C2, the second pulley group 372c and the third pulley group 373c are substantially located on the first plane C3, the second pulley group 372d and the third pulley group 373d are substantially located on the first plane C4, and the planes of the plurality of first planes C1, C2, C3, and C4 are substantially parallel to each other.

After the plurality of cables 312a, 312b, 312c, 312d, 322a, 322b, 322c, 322d, 332a, 332b, 342a, 342b, 351a, 351b are routed in the described above routing manner, the number of the guide parts of each pulley group of the plurality of second pulley groups 372a, 372b, 372c, 372d is equal to the number of the guide parts of the third pulley group on the same first plane, and at least one second pulley group has the same number of guide parts as the number of the guide parts of the third pulley group on the same first plane, so that the cables of the second segment cable bundle Z2 are distributed in layers. As shown in FIG. 12 and FIG. 15, the second segment cable bundle Z2 is divided into multi-layer sub-cable bundles Z2a, Z2b, Z2c, Z2d, and the sub-cable bundles Z2a, Z2b, Z2c, Z2d of different layers are substantially parallel to each other, so that the sub-cable bundles Z2a, Z2b, Z2c, Z2d of different layers will not interfere with each other.

The distance between the second pulley group and the third pulley group on the same first plane gradually decreases from the distal end to the proximal end of the drive device, so that the first segment cable bundle Z1 and the third segment cable bundle Z3 are located at the outside of the second guide mechanism 372 and the third guide mechanism 373, the first segment cable bundle Z1 and the third segment cable bundle Z3 are substantially parallel to each other, and the second segment cable bundle Z2 is located between the second guide mechanism 372 and the third guide mechanism 372. The second segment cable bundle Z2 is substantially perpendicular to the first segment cable bundle Z1 and the third segment cable bundle Z3, so that the first segment cable bundle Z1 and the third segment cable bundle Z3 will not cross with the sub-cable bundles Z2a, Z2b, Z2c, Z2d at different layers of the second segment cable bundle Z2, so that the first segment cable bundle Z1 and the third segment cable bundle Z3 do not affect the routing of the second segment cable bundle Z2.

Similar to the embodiment shown in FIG. 4 , after the plurality of cables 312a, 312b, 312c, 312d, 322a, 322b, 322c, 322d, 332a, 332b, 342a, 342b, 351a, 351b are guided by the second guide mechanism, each cable is rerouted on a different plane, eg, the cable segment of the first cable 312a between the first pulley 313 and the drive unit 311 and the cable segment of the first cable 312a between the first pulley 313 and the second pulley 372d are on the second plane, the cable segment of the first cable 312a between the first pulley 313 and the second pulley 372d and the cable segment of the first cable 312a between the second pulley 372d and the third pulley 373c are on a third plane, and the second plane intersects the third plane without overlapping. Therefore, the plurality of cables 312a, 312b, 312c, 312d, 322a, 322b, 322c, 322d, 332a, 332b, 342a, 342b, 351a, 351b are guided by the second guide mechanism 372 to converge to form a second segment cable bundle Z2 extending along the edge of the drive device 310, so that the elongate shaft 320 can be positioned at the edge of the drive device 310.

The plurality of first pulley groups 313, 323, 333, 343, 353 include a plurality of first coaxial pulleys 313, 323, 343 and a plurality of first split-axle pulleys 353, 333, and each of the plurality of first coaxial pulleys 313, 323, 343 shares one axle, that is, one axle has multiple guide parts, and each of the plurality of first split-axis pulleys 353 and 333 includes one axle and one guide part, that is, each guide part is individually mounted on one axle.

When the axle of the first coaxial pulley is not parallel to the axles of the plurality of second pulley groups 372a, 372b, 372c, 372d of the second guide mechanism 372, the cables guided by the same first coaxial pulley are guided by different second pulleys. As shown in FIG. 12, none of the axles of the plurality of first coaxial pulleys 313, 323, 343 are parallel to the axles of the plurality of second pulleys 372a, 372b, 372c, 372d, wherein the axle of the first coaxial pulley 343 forms an acute angle with the axles of the plurality of second pulleys 372a, 372b, 372c, 372d, the axles of the first coaxial pulleys 313 and 323 are substantially perpendicular to the axles of the plurality of second pulleys 372a, 372b, 372c, 372d. The axle 3131 of the first coaxial pulley 313 is provided with a plurality of guide parts 3132, 3133, 3134, 3135, and the cables 312d, 312b, 312a, 312c guided by the guide parts 3132, 3133, 3134, 3135 are respectively guided by the second pulleys 372a, 372b, 372c, 372d of the second guide mechanism 372, and then extend to the third pulleys 373a, 373b, 373c, 373d of the third guide mechanism 373.

As shown in FIG. 12 and FIG. 13A, when the first segment cable bundle Z1 enters the grooves of the plurality of guide parts 3722, 3723 and 3724 on the second pulley group 372a of the second guide mechanism 372, the first segment cable bundle Z1 contacts with the grooves of the plurality of guide parts 3722, 3723, 3724, and multiple contact points where the first segment cable bundle Z1 contacts the plurality of guide parts 3722, 3723, 3724 are located on the straight line r1. Similarly, the first segment cable bundle Z1 and the second pulley group 372b, 372c, 372d also have multiple contact points when they start to contact, and the contact points are shown as black dots in FIG. 13, wherein the square black dots represent fixed contact points P1, and the fixed contact points P1 are contact points between the second pulley group and the cable guided by the first coaxial pulley 313, 323, 343. A plurality of circular black dots represent adjustable contact points P2, and the adjustable contact points P2 are the contact points between the second pulley group and the cable guided by the first sub-axis pulleys 333, 353. The plurality of contact points P1 and P2 are respectively located on the straight lines r2, r3 and r4 which are substantially parallel to each other.

The positions of the plurality of second pulley groups 372a, 372b, 372c, and 372d are fixed, so the distances between the straight lines r1, r2, r3, and r4 are substantially fixed, therefore, the positions of the axles of the first coaxial pulleys 313, 323, 343 relative to the straight lines r1, r2, r3, and r4 are also fixed. If the positions of the first coaxial pulleys 313, 323, and 343 relative to the straight lines r1, r2, r3, and r4 change, It is possible that where some of the cable segments in first segment cable bundle Z1 may be not parallel to each other. For example, the included angle between the axle of the first coaxial pulley 343 and the straight line r1 is about 45°. The cable 342a is guided by a guide part of the first coaxial pulley 343 and then extends to the second pulley 372a. The contact point between the cable 342a and the second pulley 372a is located on the straight line r1, the cable 342b is guided by another guide part of the first coaxial pulley 343 and then extends to the second pulley 372b, and the contact point between the cable 342b and the second pulley 372b is located on the straight line r2. If the position of the first coaxial pulley 343 is deviated, the following two situations may occur: the contact point between the cable 342a and the second pulley 372a is deviated from the straight line r1, or the contact point between the cable 342b and the second pulley 372b is deviated from the straight line r2. Either of the above two situations will cause the cable 342a or the cable 342a to be non-parallel with other cables in the first segment cable bundle Z1, thereby affecting the efficiency of the cable transmission driving force.

By adjusting the relative positions of the sub-pulleys of the plurality of first split-axis pulleys 333 and 353, the rigid routing method can be changed, thus by adjusting the sub-pullyes of the first split-axis pulleys 333, 353, it is flexible to select which second pulley group of the second guide mechanism 372 to guide the cable. In other words, by adjusting the relative positions of the sub-pulleys of the plurality of first split-axis pulleys 333, 353, the cables can be adapted to the second pulley group with different numbers of guide parts, thereby flexibly configuring the number of cables guided by each of the second pulleys 372a, 372b, 372c, 372d. For example, the first split-axis pulley 353 includes sub-pulleys 353a, 353b, and the sub-pulley 353a and the sub-pulley 353b do not share the axle, so that the sub-pulley 353a and the sub-pulley 353b can move relatively on the horizontal plane, so that the cables 352a, 352b guided by the first split-axis pulley 353 can be guided by the second pulley groups 372b of the second guide mechanism 372, rather than requiring multiple cables guided by the coaxial pulley to be guided by different second pulley group as with the first coaxial pulley.

The first split-axis pulleys 333, 353 are disposed at the farthest end of the first guide mechanism 371, so that when adjusting the sub-pulleys of the first split-axis pulleys 333, 353, the respective sub-pulleys of the first split-axis pulleys 333, 353 do not interfere with the cables in the first segment cable bundle Z1.

FIG. 13B is an enlarged schematic view of the contact points of FIG. 13A, as shown in FIG. 13B, the cables 322c, 342a, 352a are guided by the first guide mechanism 371 and extend to the second pulley 372a of the second guide mechanism 372, and then extend to the third pulley group 373a of the third guide mechanism after being guided by the second pulley 372a of the second guide mechanism 372, so that there are three fixed contact points P1 on the straight line r1.

The cables 322a, 342b, 352b, 352a, 312b are guided by the first guide mechanism 371 and extend to the second pulley group 372b of the second guide mechanism 372, and then extend to the third pulley group 373b of the third guide mechanism 373 after being guided by the second pulley 372b, therefore there are five contact points on the straight line r2, the five contact points include three fixed contact points P1 and two adjustable contact points P2.

The cables 322b, 332a, 332b, 312a are guided by the first guide mechanism 371 and extend to the second pulley 372c of the second guide mechanism 372, and then extend to the third pulley group 373c of the third guide mechanism 373 after being guided by the second pulley group 372c, therefore, there are four contact points on the straight line r3, and the four contact points include two fixed contact points P1 and two adjustable contact points P2.

The cables 322d, 312c are guided by the first guide mechanism 371 and extend to the second pulley group 372d of the second guide mechanism 372, and then extend to the third pulley group 373d of the third guide mechanism 373 after being guided by the second pulley group 372d, so that there are two fixed contact points P1 on the straight line r4. By adjusting the first split-axis pulleys 333 and 353, the plurality of adjustable contact points P2 can be located at any position on the straight line r1 to the straight line r4.

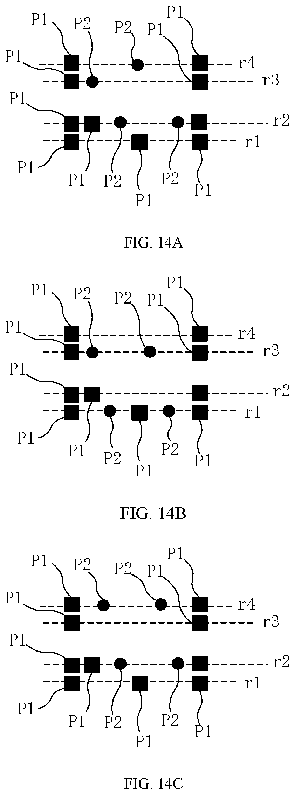

By adjusting the first split-axis pulleys 333, 353, some other distributions of contact points for cable routing modes can be obtained. As shown in FIG. 14A, each sub-pulley of the first split-axis pulley 333 can be adjusted to adapt to the situation that the second pulley group 372c and the second pulley group 372d have three guide parts, that is, there are three contact points on the straight line r3 and the straight line r4 respectively.

As shown in FIG. 14B, each sub-pulley of the first split-axis pulley 353 can be adjusted to adapt to the situation that the second pulley group 372a has five guide parts and the second pulley group 372b has three guide parts, that is, there are five contacts on the straight line r1 point and there are two contact points on the line r2.

As shown in FIG. 14C, each sub-pulley of the first split-axis pulley 333 can be adjusted to adapt to the situation that the second pulley group 372c has two guide parts and the second pulley group 372d has four guide parts, that is, there are two contact points on the line r3 and four contact points on the line r4.

As shown in FIG. 14D, each sub-pulley of the first sub-axis pulley 333 and the first sub-axis pulley 353 can be adjusted to adapt to the situation that the second pulley group 372b has 4 guide parts and the second group pulley 372d has 3 guide parts, that is, there are 4 contact points on the straight line r2 and 3 contact points on the straight line r4.

In other embodiments, the drive device 310 may, as in the embodiment shown in FIG. 7, only include the first guide mechanism 371 and the second guide mechanism 372, without including the third guide mechanism 373, therefore the elongate shaft 320 extends in a direction perpendicular to the rotation direction of the drive unit.

FIG. 15 is a side view of FIG. 12, in order to more clearly show the cable wiring of the drive device 310, part of the drive unit is hidden and some components not shown in FIG. 12 are shown in FIG. 15 . As shown in FIG. 15, after the first segment cable bundle Z1 is guided by the first guide mechanism 371, the cables in the first segment cable bundle Z1 are substantially parallel to each other, and the first segment cable bundle Z1 presents the structure of multi-layered cable bundle. That is, the first segment cable bundle Z1 includes the multi-layered sub-cable bundles Z1a, Z1b, Z1c, Z1d, and the multi-layered sub-cable bundles Z1a, Z1b, Z1c, Z1d form multi-layer sub-cable bundles Z2a, Z2b, Z2c, Z2d after being guided by a plurality of second pulleys of the second guide mechanism 372 372a, 372b, 372c, 372d.

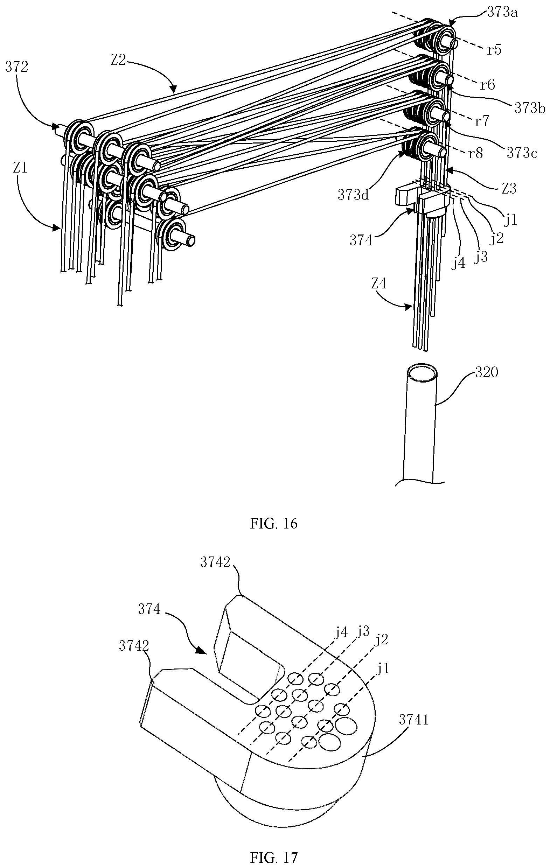

As shown in FIGS. 16 and 17, the second segment cable bundle Z2 is guided by the third guide mechanism 373 to form a third segment cable bundle Z3, and the third segment cable bundle Z3 is guided by the fourth guide mechanism 374 to form a fourth segment cable bundle Z4, the fourth segment cable bundle Z4 extends through the elongate shaft 320 to the wrist 330 and the end effector 340. The fourth guide mechanism 374 includes a body 3741, and two fixing seats 3742 extending from the body 3741 to the inside of the housing 310a. The fixing seats are configured to fix the main body 3741 on the housing 310a, the main body 3741 also has a plurality of guide holes 312a', 312b', 312c', 312d', 322a', 322b', 322c', 322d', 332a', 332b', 342a', 342b', 351a', 351b' for aplurality of cables 312a, 312b, 312c, 312d, 322a, 322b, 322c, 322d, 332a, 332b, 342a, 342b, 351a, 351b to pass through.

After being guided by the plurality of third pulley groups 372a, 372b, 372c, 372d of the third guide mechanism 373, the plurality of cables 312a, 312b, 312c, 312d, 322a, 322b, 322c, 322d, 332a, 332b, 342a, 342b, 351a, 351b leave the plurality of third pulley groups 373a, 373b, 373c, 373d, and there are a plurality of contact points respectively located on a plurality of second straight lines r5, r6, r7, r8. The plurality of second straight lines r5, r6, r7, r8 are projected onto the fourth guide mechanism 3741 to form a plurality of third straight lines j1, j2, j3, j4, and the plurality of third straight lines j1, j2, j3, j4 are substantially parallel to the axles of the plurality of second pulleys 372a, 372b, 372c, 372d. A plurality of guide holes 312a', 312b', 312c', 312d', 322a', 322b', 322c', 322d', 332a', 332b', 342a', 342b', 351a', 351b' are arranged respectively on the plurality of third straight lines j1, j2, j3, and j4, that is, the number of rows of the plurality of guide holes is equal to the number of the third pulley group.

The arrangement of the guide holes on each of the plurality of third straight lines j1, j2, j3, and j4 is substantially the same as the arrangement of the guide parts on each of the plurality of second straight lines r5, r6, r7, and r8. That is, the number of guide holes on each of the plurality of third straight lines j1, j2, j3, and j4 is equal to the number of guide parts on each of the plurality of second straight lines r5, r6, r7, and r8. The distance between the guide holes on each of the third straight lines j1, j2, j3, j4 is substantially equal to the distance between the guide parts on each of the plurality of second straight lines r5, r6, r7, r8, so that the third segment cable bundle Z3 is still a layered wiring mechanism, and the number of layers of the third segment cable bundle Z3 is equal to the number of layers of the second segment cable bundle Z2, and the number of cables on the corresponding layers of the third segment cable bundle Z3 and the second segment cable bundle Z2 is equal.

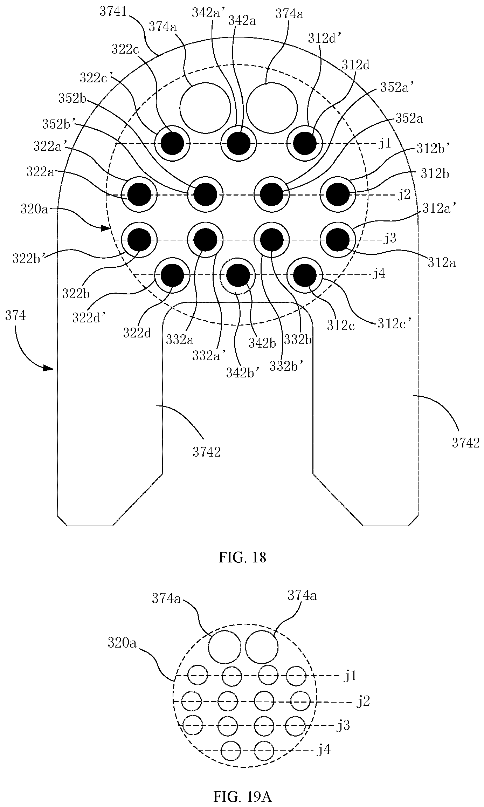

Specifically, as shown in FIG. 15, the third segment cable bundle Z3 includes multi-layer sub-cable bundles Z3a, Z3b, Z3c, Z3d which are substantially parallel to each other. As shown in FIGS. 16 and 18, three cables 322d, 342b, 312c in the sub-segment cable bundle Z3a are guided by three guide parts on the third pulley 373a of the third guide mechanism 373 and then pass through the guide holes 322d', 342b', 312c' arranged on the third straight line J1, four cables 322b, 332a, 332b, 312a in the sub-cable bundle Z3b are guided by four guide parts on the third pulley 373b, and then pass through the guide holes 322b', 332a', 332b', 312a' arranged on the third straight line J2, four cables 322a, 352b, 352a, 312b in the sub-cable bundle Z3c pass through the guide holes 322a', 352b', 352a', 312b' arranged on the third straight line J3 after being guided by four guide parts on the third pulley 373c, three cables 322c, 342a, 312d in the sub-cable bundle Z3d are guided by the four guide parts on the third pulley 373c and then pass through the guide holes 322c', 342a', 312d' arranged on the third straight line J4. A plurality of cables 312a, 312b, 312c, 312d, 322a, 322b, 322c, 322d, 332a, 332b, 342a, 342b, 351a , 351b pass through the plurality of guide holes 312a', 312b', 312c', 312d', 322a', 322b', 322c', 322d', 332a', 332b', 342a', 342b', 351a', 351b' and then enter the elongate shaft 320, and finally extend to the wrist 330 and the end effector 340.

Since the number of guide holes on each of the plurality of third straight lines j1, j2, j3, j4 is the same as the number of guide parts on each of the plurality of second straight lines r5, r6, r7, r8, so that the cables passing through the plurality of guide holes on one of the plurality of third straight lines j1, j2, j3, and j4 can be one-to-one corresponding to the third pulley, so that the plurality of cables 312a, 312b, 312c, 312d, 322a, 322b, 322c, 322d, 332a, 332b, 342a, 342b, 351a, 351b are guided by the fourth guide mechanism 374, the third guide mechanism 373, the second guide mechanism 372 and the first guide mechanism 371 in sequence to be easily assembled to a plurality of drive units 311 , 321 , 331 , 341,351.

Specifically, when the cables 322c, 342a, 312d are assembled, the proximal ends of the cables 322c, 342a, 312d pass through the guide hole 322c', 342a', 312d' on the third straight line j1 at the edge of the main body 374 on the side away from the mounting seat 3742, and extend to the third pulley group 373a of the third guide mechanism 373. Since the positions of the guide holes 322c', 342a', 312d' on the main body 374 are obviously different from the positions of other guide holes, during assembly, the cables 322c, 342a, 312d passing through the guide holes 322c', 342a', 312d' will not be confused with other cables. When the cables 322c, 342a, 312d are assembled to the third pulley group 373a of the corresponding third guide mechanism 373, since the position of the third pulley 373a and the number of its guide parts are uniquely corresponding to the guide holes 322c', 342a', 312d', the cables 322c, 342a, 312d can be assembled to the third pulley group 373a without error, thus the error of fitting the cables 332d, 342b, 312c to other pulleys can be avoided.

Since the second pulley group 372a of the second guide mechanism 372 and the third pulley group 373a are located on the same first plane and have the same number of guide pulleys, the corresponding relationship is unique, so it is very convenient and accurate to assemble the cables 322c, 342a, 312d from the third pulley 373a to the second pulley 372a, in this process, the error of fitting one of the cables 322c, 342a, 312d to the other second pulley group can be avoided.

In the process of assembling the cables 322c, 342a, 312d from the second pulley 372a to the first guide mechanism 371, as shown in FIG. 13A, since the cables 322c, 342a, 312d are located on the outermost layer of the first segment cable bundle, the cables 322c, 342a, 312d only need to be assembled to the guide parts, farthest from the third guide mechanism 373, of the first pulley groups 323, 343, 313 of the first guide mechanism 371, thus the sub-cable bundle Z1a is formed, thereby avoiding an error in assembling the cables 322c, 342a, 312d to other first pulley groups, and the method for assembling cables of other layers is the same, and will not be repeated here. The cable bundle structure for layered wiring and the method of installing cables in layers can improve the accuracy and efficiency of assembling cable.

The main body 3741 of the fourth guide mechanism 374 also includes a plurality of through holes 374a with different diameters from the diameters of the guide holes. The plurality of through holes 374a are used for other forms of wires other than cables to pass through, such as the plurality of through holes 374a are used for power supply wires to pass through, or wires for transmitting image data to pass through. A plurality of through holes 374a and a plurality of guide holes 312a', 312b', 312c', 312d', 322a', 322b', 322c', 322d', 332a', 332b', 342a', 342b', 351a', 351b ' are divided into multiple rows and distributed in a circular area 320a on the main body 3741. The area of the circular area 320a is substantially equal to the area of the proximal opening of the elongate shaft 320, so that the plurality of cables 312a, 312b, 312c, 312d , 322a, 322b , 322c , 322d , 332a, 332b , 342a, 342b , 351a, 351b are concentrated in the circular area 320a and pass straight through a plurality of guide holes into the elongate shaft 320.

As shown in FIG. 18, a plurality of through holes 374a are arranged on the edge of the main body 3741 on the side away from the mounting seat 3742. Generally, the diameter of the through holes 374a is greater than the radius of the guide hole, and the space on the main body 3741 occupied by one of the through holes 374a is greater than the space occupied by one guide hole, and arranging the through hole 374a at the edge of the main body 3741 can reduce the influence on the positioning of the guide hole. There is a greater space in the middle area of the circular area 320a, i.e., a space near the third straight line j2 is greater, and at the edge of the circular area 320a, i.e., a space near the third straight lines j 1 and j4 is smaller. Therefore, both the third straight line j2 and the third straight line j3 have four guide holes, and both the third straight line j 1 and the third straight line j4 have three guide holes, so that more space on the main body 3741 can be provided for the two through holes 374a.

The distribution of the through holes 374a and the guide holes of the fourth guide mechanism 374 according to an embodiment of the present disclosure is shown in FIG. 19A, four guide holes are distributed on each of the third straight lines j1 , j2 , and j3 , and the third straight line j4 at the edge of the main body of the fourth guide mechanism 374 is distributed with only two guide holes, so that the guide holes on the third straight line j4 can be offset more towards the edge of the main body of the fourth guide mechanism, so that there is more space for two through holes 374a at the edge of the other side of the main body to make the through-holes 374a greater in diameter, thereby allowing wires or data lines with greater diameters to pass through.

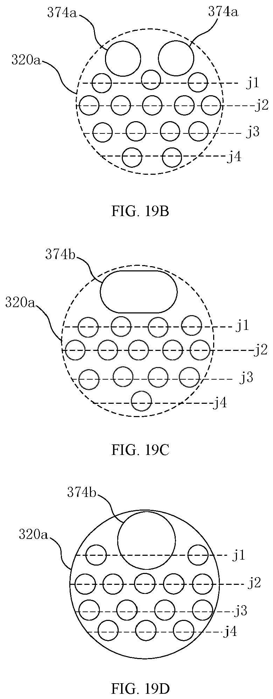

The distribution of the through holes 374a and the guide holes of the fourth guide mechanism 374 according to an embodiment of the present disclosure is shown in FIG. 19B, the number of guide holes arranged on the third straight lines j1, j2, j3, j4 are 3, 5, 4, 2, respectively. Compared with the embodiment shown in FIG. 19A, in this embodiment, the guide holes on the third straight line j1 in the circular region 320a is fewer, so that the two through holes 374a can have greater diameters.

In some other embodiments of the present disclosure, the distribution of the through holes 374b and the guide holes of the fourth guide mechanism 374 is shown in FIG. 19C and FIG. 19D. There is only one through hole 374b in each of FIGS. 19C and 19D. Generally, the through hole 374b is configured for a data wire or an optical cable for transmitting image data to pass through, and the through-hole 374b has a greater cross-sectional area than that of the through-hole 374a in the above-described embodiment. As shown in FIG. 19C , the number of guide holes arranged on the third straight line j1, j2, j3, j4 are 4, 5, 4, and 1, respectively. Since only one guide hole is distributed on the third straight line j4, there is more space provided for the through hole 374b at the edge of the main body of the fourth guide mechanism on the side away from the third straight line j4, so that the through hole 374b can have a greater cross-sectional area. As shown in FIG. 19D , the number of guide holes arranged on the third straight line j1, j2, j3, and j4 are 2, 5, 4, and 3, respectively, and the through hole 374b is located between the two guide holes on the third straight line j1, so that the through hole 374b can have a greater cross-sectional area.

A tensioning device is provided between the plurality of drive units 311, 321, 331, 341, 351 and the first guide mechanism 317, and the tensioning device is configured to tension the plurality of cables 312a, 312b, 312c, 312d, 322a, 322b, 322c, 322d, 332a, 332b, 342a, 342b, 351a, 351b, the tensioning device includes a housing and a plurality of first tensioning mechanisms 380 mounted in the housing and/or a plurality of second tensioning mechanisms 390 mounted in the housing. A plurality of first tensioning mechanism 380 is provided between the first drive units 311, 321 and the first guide mechanism 317, and a plurality of second tensioning mechanisms are provided between the second drive units 331, 341, 351 and the first guide mechanism 317.

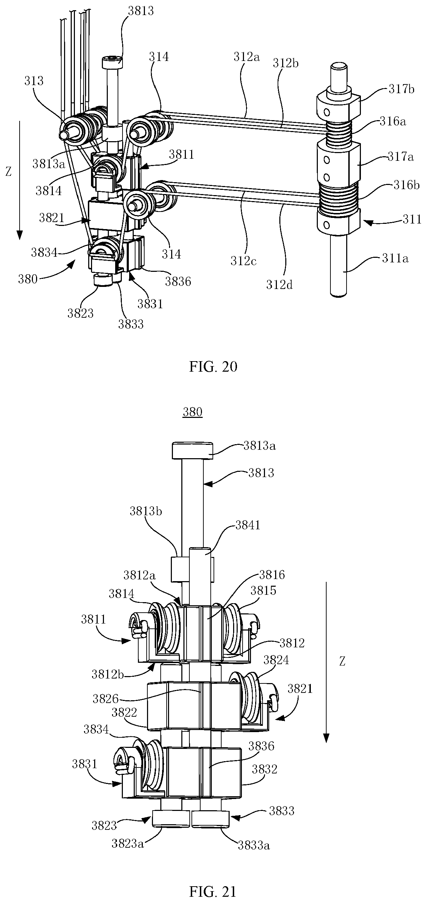

The first tensioning mechanism of an embodiment of the present disclosure is shown in FIGS. 20-22. The first tensioning mechanism 380 is disposed between the first drive unit 311 and the first pulley group 313 of the first guide mechanism 317. The first tensioning mechanism 380 is configured to tension the first cables 312a, 312b, the second cable 312c and the third cable 312d. The first tensioning mechanism 380 includes a first tensioning member 3821, a second tensioning member 3811, a third tensioning member 3831 and a resisting portion. The second tensioning member 3811 is used to simultaneously tension the first cable 312a and the first cable 312b, the first tensioning member 3821 and the third tensioning member 3831 are used to tension the second cable 312c and the third cable 312d, respectively. In other embodiments, in case that there is no need to tension more cables, the first tensioning mechanism 380 may also include only the first tensioning member 3821 and the second tensioning member 3811, or only the first tensioning member 3821.

The first tensioning member 3821 includes a first tension block 3822 and a first push rod 3823, a first guide part 3824 is provided on the first side of the first tension block 3822, and the second cable 312c is guided by a holding mechanism 314, then it extends to the first pulley block 313 after being guided by the first guide part 3824. The first guide part 3824 is located between the cable segment of the second cable 312c and the resisting portion, the above-mentioned cable segment is located between the first pulley block 313 and the holding mechanism 314. The first push rod 3823 is screwed with the first tension block 3822 and passes through the first tension block 3822 to abut against the resisting portion. Rotating the first push rod 3823 can drive the first tension block 3822 in the first direction (Z direction of Fig. 20) or in a second direction opposite to the first direction to move, so that the first guide part 3824 on the first tension block 3822 drives the cable segment of the cable 312c between the holding mechanism 314 and the first pulley group 313 to move between the distal and proximal ends of the drive device 310, thereby tensioning or slackening the cable 312c.

The second tensioning member 3811 includes a second tension block 3812 and a second push rod 3813, the first push rod 3823 and the second push rod 3813 are arranged opposite to each other, and the resisting portion is provided between the first push rod 3823 and the second push rod 3813. A second guide part 3814 and a third guide part 3815 are respectively provided on two first side surfaces of the second tension block 3812, and the first cables 312, 312b are guided by the holding mechanism 314 and then are guided by the second guide part 3814 and the third guide part 3815 respectively, and then guided by the first pulley group 313 of the first guide mechanism317. In some other embodiments, the second tensioning member may only include one guide part for guiding one cable, and in this case, the second tensioning member is used for tensioning one cable.

A sleeve 3813b is installed on a housing of the first tensioning mechanism 380, the sleeve 3813b is threaded with the second push rod 3813, and the second tension block 3812 has a first end 3812a away from the first tension block 3822 and a second end 3812b opposite to the first end 3812a and close to the first tension block 3822. The end of the second push rod 3813 abuts against the first end 3812a of the second tension block 3812. The adjusting end 3813a of the second push rod 3813 opposite to its end is rotated in the tensioning direction, to cause the second push rod 3813 push the second tension block 3812 to move in the first direction, so that the second guide part 3814 and the third guide part 3815 part on the second tension block 3812 respectively drive the cable segments of the first cables 312a, 312b between the holding mechanism 314 and the first pulley group 313 to move in a first direction for tensioning the first cables 312a, 312b. The above-mentioned tensioning direction refers to rotating the push rod in this direction so that the tension block driven of the push rod tensions the cable. In some other embodiments, the second push rod may also cooperate with the second tension block through threads.

When the adjusting end 3813a is rotated in an opposite direction to the tensioning direction, the second push rod 3813 moves in the second direction opposite to the first direction, thereby releasing the second tension block 3812. Since the first cable 312a,312b itself have a certain tension, so that the second tension block 3812 is driven to move in the second direction by the tension force of the first cables 312a, 312b themselves, so that the first cables 312a, 312b are slackened.

The third tensioning member 3831 includes a third tension block 3832 and a third push rod 3833. A fourth guide part 3834 is provided on the first side of the third tension block 3832, and the fourth guide part 3834 is provided between a resisting portion on the opposite side of the first guide part 3824 and the third cable 312d. The third cable 312d is guided by the holding mechanism 314 and then guided by the fourth guide part 3834 to extend to the first pulley group 313, the third push rod 3833 fits with the third tension block 3823 through threads and passes through the third tension block 3832 to abut against the resisting portion. Rotating the third push rod 3833 can drive the third tension block 3823to move in the first direction or the second direction, so that the first guide part 3834 on the third tension block 3832 drives the cable segment of the third cable 312d between the holding mechanism 314 and the first pulley group 313 to move between the distal end and the proximal end of the drive device 310, thereby tensioning or slackening the cable 312d.

The first tensioning mechanism 380 further includes a guide protrusion 386 and a guide post 3841 for guiding the movement of the first tension block 3822, the second tension block 3812 and the third tension block 3833. The guide protrusion 386 includes a second protrusions 3816, a first protrusions 3826 and a third protrusion 3836 respectively located on the second sides of the second tension block 3812, the first tension block 3822 and the third tension block 3833, and groove defined in the housing of the first tensioning mechanism 380 and configured to cooperate with the second protrusion 3816, the first protrusion 3826 and the third protrusion 3836. When the second tension block 3812, the first tension block 3822 and the third tension block 3833 move in the first direction or the second direction, the second protrusion 3816, the first protrusion 3826 and the third protrusion 3836 slide in the groove to guide the second tension block 3812, the first tension block 3822 and the third tension block 3833 to move.

The guide post 3841 passes through the through holes of the first tension block 3822, the second tension block 3812 and the third tension block 3833. During movement between a proximal end and distal end of the tensioning device, the second tension block 3812, the first tension block 3823 and the third tension block 3833 slide up and down along the guide post 3841.

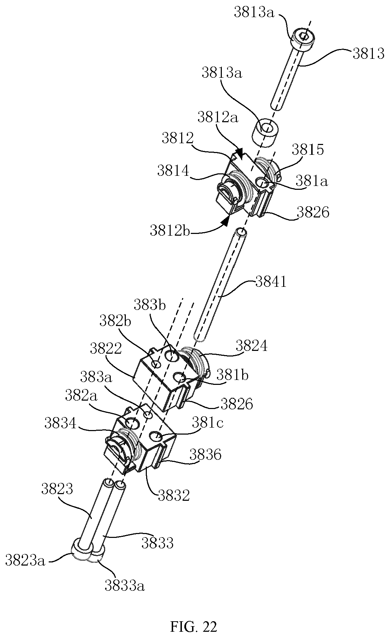

As shown in FIG. 22, the first tension block 3822, the second tension block 3812 and the third tension block 3833 have a plurality of holes, and the resisting portion in this embodiment is the second end 3812b of the second tension block 3812. The second end 3812b of the second tension block 3812 is used as a resisting portion to make the mechanism of the first tensioning mechanism 380 more compact. The end of the first push rod 3823 passes through a first hole 382a on the third tension block 3833 and a second hole 382b on the first tension block 3822 and then abuts against the second end 3812b of the second tension block 3811. The proximal end of the third push rod 3833 passes through a third hole 383a on the third tension block 3833 and a fourth hole 383b on the first tension block 3822 and then abuts against the second end 3812b of the second tension block 3811. The guide post 3841 passes through a fifth hole 381a of the second tension block 3811, a sixth hole 381b of the first tension block 3822, and a seventh hole 381c of the third tension block 3832, so as to make the second tension block 3811, the first tension block 3822 and the third tension block 3832 can slide up and down along the guide post 3841.

The second hole 382b of the first tensioning member 3821 has internal threads to cooperate with the external threads on the first push rod 3823. When the cable 312c is tensioned, the adjusting end 3823a at the distal end of the first push rod 3823 is rotated in the tensioning direction. Since the end of the first push rod 3823 abuts against the second end 3812b of the second tension block 3811, the first push rod 3823 cannot move in the second direction because it is resisted by the second end 3812a, thus forcing the first tension block moves in a first direction to tension the cable 312c.

Since the second cable 312c itself has a certain tensioning force, when the adjusting end 3823a at the distal end of the first push rod 3823 is rotated in a direction opposite to the tensioning direction, the first tension block 3822 moves in the second direction under the tension force of the second cable 312c, so that the second cable 312c is slackened.

During the movement of the first tension block 3822 in the first direction or the second direction, the first tension block 3822 is double guided, that is, the first protrusion 3826 of the first tension block 3822 moves in the groove in the housing, and the first tension block 3822 moves up and down along the guide post 3841, so that there is no deviation of the movement track during the movement of the first tension block 3822 in the first direction or the second direction.

The third hole 383a of the third tensioning member 3833 has internal threads to fit with the external threads on the third push rod 3833, and a distal end of the third push rod 3833 abuts the second end 3812b of the second tension block 3811. When the cable 312d need to be tensioned or slackened, rotating the adjusting end 3833a at the distal end of the third push rod 3833 can make the third protrusion 3836 of the third tensioning member 3833 move in the groove in the housing, and the third tension block 3832 move up and down along the guide post 3841 to tension or slacken the third cable 312d. The method for tensioning or slackening the third cable 312d using the third tensioning member 3833 is the same as the method for tensioning or slackening the second cable 312c using the first tensioning member 3823 described above, and will not be repeated here.

Since the adjusting ends of the second tensioning member 3811 is opposite to the adjusting ends of the first tensioning member 3821 and the third tensioning member 3823, that is, the adjusting end 3813a of the second tensioning member 3811 is located at one side of the first end 3812a of the second tension block 3812, and the adjusting end 3823a of the first tensioning member 3821 and the adjusting end 3833a of the third tensioning member 3831 are located on one side of the second end 3812b of the second tension block 3812. The second push rod 3813, the first push rod 3823 and a third push rod 3833 are staggered from each other, so that the entire first tensioning mechanism 380 is arranged along the axial direction of the push rod. The space in the longitudinal direction (the axial direction of the push rod) occupied by the first tensioning mechanism 380 is greater than the space occupied in the lateral direction and can make the lateral volume of the entire drive device 380 smaller, so that when a plurality of surgical instruments 300 are gathered together as shown in FIG. 6, the overall lateral volume is smaller.

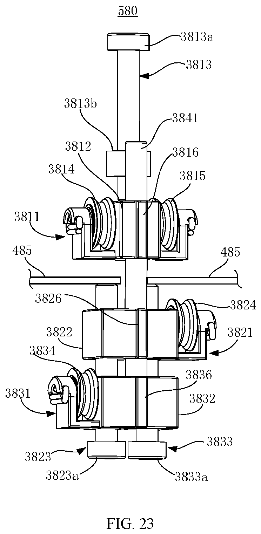

The first tensioning mechanism of another embodiment of the present disclosure is shown in FIG. 23. The difference between the first tensioning mechanism 480 in this embodiment and the first tensioning mechanism 380 shown in FIG. 21 is that the resisting portion in this embodiment is a baffle plate 485 arranged between the first tensioning member 3821 and the second tensioning member 3811, and the baffle plate 485 is fixedly connected with the housing of the first tensioning mechanism 480. The ends of the first push rod 3823 and the third push rod 3833 abut against the baffle plate 485. When the second cable 312c and the third cable 312d are tensioned, the baffle plate 485 prevents the first push rod 3823 and the third push rod 3833 from being moved in the second direction, whereby the first tension block 3822 and the third tension block 3832 will move in the first direction to cause the second cable 312c and the third cable 312d to be tensioned. Since the ends of the first push rod 3823 and the third push rod 3833 do not abut against the second tension block 3812, adjusting the movement of the second tension block 3812 will not affect the first tension block 3822 and the third tension block 3823, the movements of the three tensioners are independent of each other so that the cables tensioned by the three tensioning members are also tensioned independently of each other.

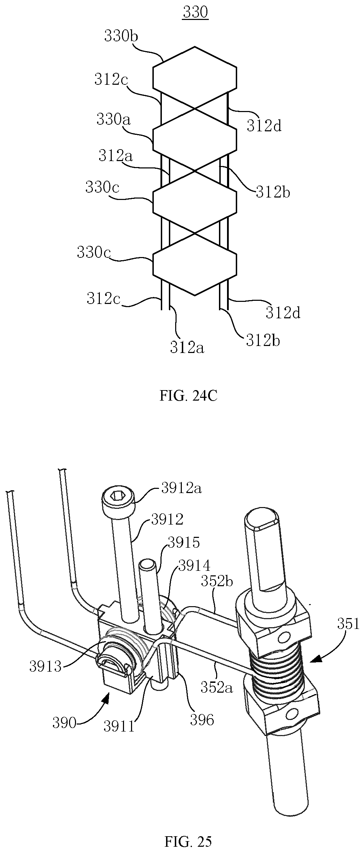

The tensioning device further includes a plurality of second tensioning mechanisms. The second tensioning mechanisms 390 are used to tension two cables at the same time. The second tensioning mechanisms 390 are equivalent to the second tensioning members 3811 of the first tensioning mechanism 380. As shown in FIG. 25, the second tensioning mechanism 390 includes a fourth tension block 3911 and a fourth push rod 3912, the end of the fourth push rod 3912 abuts the fourth tension block 3911, The movement of the second tension block 3911 is guided by the guide protrusions 396 fitting with the grooves in the housing of the tensioning device, and the guiding posts 3914.

Both sides of the fourth tension block 3911 are provided with a fifth guide part 3913 and a sixth guide part 3914 for guiding the cables 352a, 352b, respectively, and the cables 352a, 352b can be tensioned or slackened by rotating the adjusting end 3912a of the fourth push rod 3912. The specific tension process is the same as the tension process of the second tensioning member 3811 of the first tensioning mechanism 380 described above, and will not be repeated here.

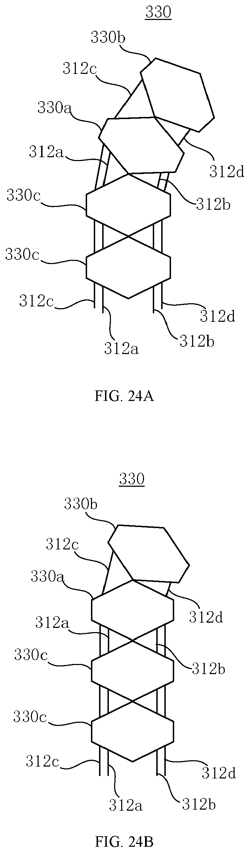

As shown in FIG. 24A, the distal ends of the first cables 312a, 312b are connected to the first joint 330a of the wrist 330, the first drive unit 311 controls the rotation of the first joint 330a through the first cables 312a, 312b. The distal ends of the second cable 312c and the third cable 312d are respectively connected to two sides of the second joint 330b, and the first drive unit 311 controls the rotation of the second joint 330b through the second cable 312c and the third cable 312d.

In the process of assembling the surgical instrument 300, there may be a case that the first joint 330a and the second joint 330b are not in a straight posture (i.e, the zero position) after adjusting the tension of the first cables 312a, 312b, the second cable 312c and the third cable 312d.

As shown in FIG. 24A, the third joint 330c of the wrist 330 is in a straight posture, and the first joint 330a and the second joint 330b are in a yaw posture after rotating a certain angle relative to the straight posture. Therefore, the first joint 330a and the second joint 330b need to be adjusted back to the straight posture after the cables is tensioned, so that before the surgical instrument 300 is operated, the wrist 330 and the end effector 340 of the surgical instrument 300 are in the zero position, so that the control of the surgical instrument 300 is more precise.

Therefore, the present disclosure also provides a method for tensioning, which is suitable for the first tensioning mechanism 380 to tension the four cables 321a, 312b, 312c, 312d from the first drive unit 311, and the tensioning method is as below:
First, adjust the second push rod 3813, the first push rod 3823 and the third push rod 3833, so that the second tensioning member 3811 tensions the first cable 312a and the first cable 312b at the same time, the first tensioning member 3821 and the third tensioning member 3831 tensions the second cable 312c and the third cable 312d, respectively.

Then, the first drive unit 311 is rotated and adjusted, so that the first drive unit 311 can adjust the first joint 330a to a straight posture through the first cables 312a and 312b. Finally, by adjusting the first push rod 3823 individually, the first tensioning member 3821 can tension or slacken the second cable 312c, and/or, by individually adjusting the third push rod 3833, the first tensioning member 3821 can tension or slacken the third cable 312c, so that the second joint 330b is adjusted to a straight posture.

Specifically, firstly adjust the first drive unit 311 by rotating, so that the first drive unit 311 pulls the first cable 312a and releases the first cable 312b at the same time, so that the first joint 330a rotated leftwards from the position shown in FIG. 24A to the position aligned with the third joint 330c shown in FIG. 24B by rotation to left, that is, the first joint 330a is adjusted to a straight posture.

Although the first joint 330a has been adjusted to the straight posture after the previous step, the second joint 330b has not been adjusted to the straight posture at this time, since the first capstan 316a and the second capstan 316b are integrally formed, the posture of the joint 330b can not be adjusted through rotating and adjusting the first drive unit 311, if the first drive unit 311 is rotated again, the first cables 312a, 312b will be pulled or released again while the second cable 312c and the third cable 312d are pulled or released, so that the first joint 330a that has been adjusted to the straight posture is rotated to the non-straight posture again.

In order to solve this problem, after the first joint 330a is adjusted to a straight posture by adjusting the first drive unit 311, the second joint 330b is adjusted to be straight by adjusting the first tensioner 3821 and/or the third tensioner 3831. Specifically, the second joint 330b can be adjusted to a straight posture through the following three adjustment methods:
(1) Rotating and adjusting the first push rod 3823 alone to make the first tension block 3822 move in the first direction, causing the first tension block to further tension the second cable 312c, so that the tension of the second cable 312c is greater than that of the third cable 312d, the second cable 312c pulls the second joint 330b to rotate leftward, so that the second joint 330b rotates leftward from the yaw posture shown in FIG. 24B to the straight posture shown in FIG. 24C.
(2) Rotating and adjusting the third push rod 3833 alone to make the third tension block 3832 move in the second direction opposite to the first direction, causing the third tension block 3832 to slacken the third cable 312d. Since the third cable 312d is slackened, the tension force of the second cable 312c is greater than the tension force of the third cable 312d, the second cable 312c will pull the second joint 330b to rotate leftward, so that the second joint 330b rotates leftward from the yaw posture shown in FIG. 24B to the straight posture shown in Figure 24C.
(3) Rotating and adjusting the first push rod 3823 to make the first tension block 3822 move in the first direction, causing the first tension block 3822 to further tension the second cable 312c, and rotating and adjusting the third push rod 3833 to make the third tension block 3832 move in the second direction, causing the third tension block 3832 to slacken the third cable 312d, so that the tensioning force of the second cable 312c is greater than the tensioning force of the third cable 312d, and the second cables 312c pull the second joint 330b to rotate leftward, so that the second joint 330b rotates leftward from the yaw posture shown in FIG. 24B to the straight posture shown in FIG. 24C .

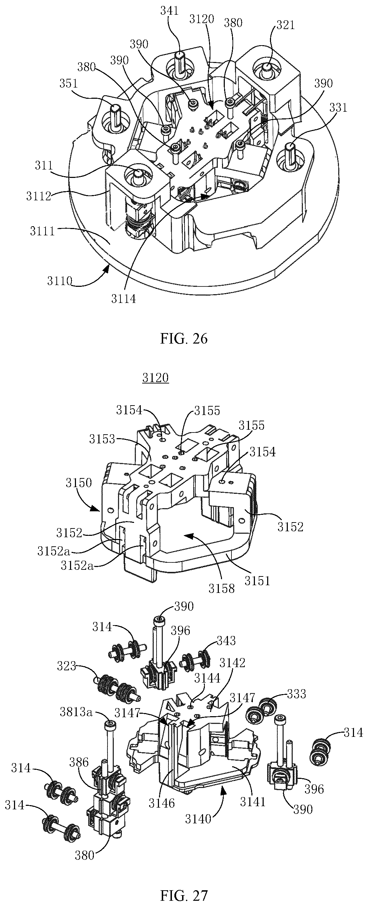

A drive device according to an embodiment of the present disclosure is shown in FIG. 26. The drive device 310 includes a base 3110 and a guide assembly 3120. The base 3110 includes a base body 3111 and amounting portion 3112 extending from the base body 3111. The plurality of drive units 311, 321, 331, 341 and 351 are installed in a mounting portion 3112, an accommodating cavity 3114 is formed in the middle area of the mounting portion 3112, and the guide assembly 3120 is installed in the accommodating cavity 3114.

The guide assembly 3120 includes a housing and a first guide mechanism 370 installed in the housing, a holding mechanism 314 and a plurality of the above-mentioned tensioning mechanisms, a plurality of cables 312a, 312b, 312c, 312d, 322a, 322b, 322c, 322d, 332a, 332b, 342a, 342b, 351a, 351b are first guided by the holding mechanism 314, then guided by the tensioning device, and finally guided by the first guide mechanism 371 to converge into the first segment cable bundle Z1. Since the first guide mechanism 370, the holding mechanism 314 and the above-mentioned multiple tensioning mechanisms are integrated into one guide assembly 3120, the plurality of cables 312a, 312b, 312c, 312d, 322a, 322b, 322c, 322d, 332a, 332b, 342a, 342b, 351a, 351b can be tensioned and guided into a cable bundle after passing through the guide assembly 3120, which saves the space of the drive device 310 and makes the structure of the entire drive device 310 more compact.

For the convenience of showing the internal structure of the guide assembly 3120, some components of the first guide mechanism 370, the tensioning mechanism and the holding mechanism 314 in FIG. 26 are not shown. As shown in FIG. 26, the housing of the guide assembly 3120 includes a first housing 3150 and a second housing 3140, and the second housing 3140 is embedded in the first housing 3130. The first housing 3150 includes a first body 3151, a side wall 3152 and a top 3153, the side wall 3152 extends from the first main body 3151 to the proximal end, the proximal end of each side wall 3152 is connected by the top 3153, a plurality of side walls 3152 are arranged at intervals, and there are gaps 3158 between the plurality of side walls 3152, through which the tensioning mechanism is mounted to the housing of the guide assembly 3120. In some other embodiments, the first casing 3150 and the second casing 3140 are integrally formed.

The second housing 3140 includes a second main body 3140, and a central post 3142 extending from the second main body 3140 to the proximal end. The central post 3142 is embedded in the cavity formed by the side wall 3132 and the top 3133 of the first housing 3130, the edge of the second body 3140 abuts the first body 3131.

The first pulley groups 323, 313 at the distal end of the first guide mechanism 370 are installed in the top 3153 of the first housing 3150, and the first pulley groups 333, 353 at the proximal end of the first guide mechanism 370 are installed in the center column 3142 of the second housing 3140. The first pulley group 343 in the middle position of the first guide mechanism 370 is installed at the middle position between the first pulley group 323 and the first split-axis pulley group 333 on the central column 3142, so that the plurality of first pulley groups 313, 323, 333, 343, 353 are located on three different horizontal planes, wherein two adjacent first pulley groups among the plurality of first pulleys 313, 323, 333, 343, 353 are located on different horizontal planes.

The holding mechanism 314 is installed on the side wall 3132 of the first housing 3150, and the holding mechanism 314 is used for keeping the cable between the drive unit and the holding mechanism in a state of constant length during tensioning cable process of the tensioning device, i.e., the direction of the cable between the drive unit and the holding mechanism is maintained during the tensioning of the cable. In this embodiment, the holding mechanism 314 is a plurality of pulley groups 314. In other embodiments, the holding mechanism 314 may also be other holding elements, such as a plurality of shafts. In some other embodiments, the guide assembly 3120 may only include the first guide mechanism 371 and the tensioning device, in this situation, the push rod of the tensioning device is perpendicular to the axis of the drive unit, and the holding mechanism may not be provided in this situation.

The tensioning device is installed between the side wall 3132 of the first housing 3150 and the central column 3142 of the second housing 3140, and the inner side of the side wall 3152 of the first housing 3150 includes a first groove 3156 and a second groove 3157, the outer side wall of the central column 3142 of the second housing 3140 has a plurality of central grooves 3146 matching the first grooves 3156 and the second grooves 3157. The guiding protrusion 386 of the first tensioning mechanism 380 is received in the center groove 3146 and the first groove 3156, and when the first tensioning mechanism 380 tensions the cable, the first tensioning mechanism 380 guides the protrusion 386 to slide in the first groove 3156 and the center groove 3146.

The guide protrusions 396 of the second tensioning mechanism 390 are accommodated in the second grooves 3157 and the central groove 3146. When the second tensioning mechanism 390 tensions the cable, the guiding protrusions 396 of the second tensioning mechanism 390 slides inside the second groove 3157 and the central groove 3146.

The top 3153 of the first housing 3150 has a plurality of tensioning holes 3154 and a plurality of wire holes 3155, and the top end of the proximal end of the central column 3142 has a plurality of wire holes 3144. The cables guided by the first guide mechanism 371 pass through the wire holes 3144 and 3155, and then converge to form the first segment cable bundle Z1.

There are grooves 3147 on both sides of the central groove 3146 of the guide assembly 3120. The grooves 3147 are used to guide the cable to extend from the first pulley group to the second guide mechanism 372. The groove bottom of the groove 3147 has a certain slope from the distal end to the top of the central column 3142, that is, the bottom of the groove near the top of the central column 3120 has a certain angle with the central axis of the central column 3142, so that the cable is close to the central axis of the central column 3142 after passing through the groove 3147, so that the first cable segments in the first segment cable bundle Z1 are closely spaced.

In one embodiment, a resting portion of the first tensioning mechanism is a side wall and/or a baffle extends from a side surface of the center column, the baffle is arranged between the second tensioning member and the first tensioning member, and the end of the push rod abuts the baffle, to prevent the movement of the second push rod and the first push rod in the second direction when tensioning the cables.

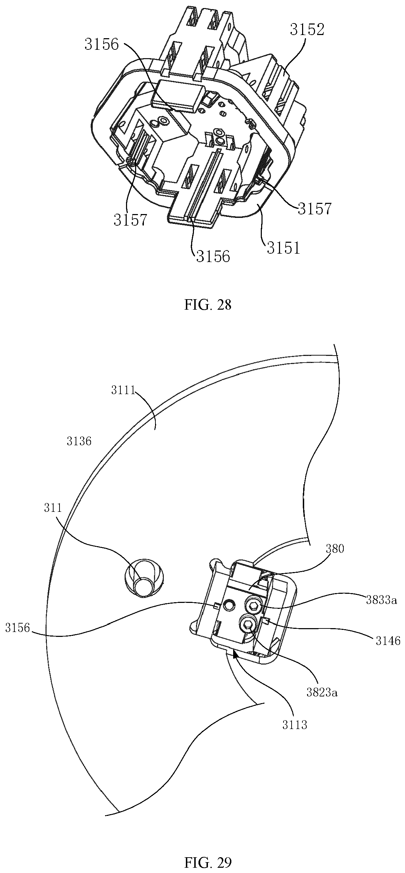

As shown in FIG. 28, the bottom of the base body 311 has a tensioning opening 3113, and the first groove 3156 and the central groove 3146 of the guide assembly 3120 extend through the tensioning opening 3113 to the distal end of the base body 311, so that the first tension block 3822 and the third tension block 3823 of the first tensioning mechanism 380 can move a greater distance along the first groove 3156 and the central groove 3146 toward the distal end of the guide assembly 3120. The first push rod 3823 and the third push rod 3833 are respectively connected to the first tension block 3822 and the third tension block 3823 through the tension hole 3113, and the second adjustment end 3823a and the third adjustment end 3833a are located at the bottom of the base body 3111.

Opposite to the first push rod 3823 and the third push rod 3833, the second push rod 3813 of the first tensioning mechanism 380 and the second tensioning mechanism 390 abuts against the second tension block 3812 through the tensioning hole 3154. The adjusting end 3813a of the second push rod 3813 is located on the top 3153 of the first housing 3150, so that the first tensioning mechanism 380 is arranged oppositely, that is, the adjusting end 3813a of the second push rod 3813 is disposed on the top of the guide assembly 3120, and the adjusting ends 3823a and 8833a of the first push rod 3823 and the third push rod 3833 are arranged at the bottom of the guide assembly 3120, so that the first tensioning mechanism 380 and the second tensioning mechanism 390 are elongated, reducing the lateral volume of the entire guide assembly 3120. At the same time, the tensioning mechanism can be adjusted from the bottom of the machine base to avoid adjusting the three tensioning parts from one side, which increases the convenience of tensioning adjustment.

The above-described embodiments have only expressed several embodiments of the present disclosure, which are described in more detail and detail, but are not therefore to be construed as limiting the scope of the present disclosure. It should be noted that variations and modifications may be made to those of skill in the art without departing from the spirit of the present disclosure, all of which fall within the scope of the present disclosure. Therefore, the scope of the appended claims should be accorded the broadest interpretation so as to encompass all such modifications and similar arrangements.

## Claims

1. A surgical instrument, comprising a drive device , a plurality of cables, an elongate shaft and an end instrument located at a distal end of the elongate shaft, the plurality of cables being connected between the drive device and the end instrument, wherein, the drive device comprises:
a plurality of drive units, the plurality of cables are connected, at an end, to the plurality of drive units;
a first guide mechanism and a second guide mechanism, wherein the plurality of cables form a cable bundle after being guided by the first guide mechanism, the cable bundle comprises a first segment cable bundle arranged between the first guide mechanism and the second guide mechanism, and a second segment cable bundle arranged between the second guide mechanism and a proximal end of the elongate shaft, the first segment cable bundle extends to the second guide mechanism in a first direction, and the second segment cable bundle extends toward the proximal end of the elongate shaft in a second direction different from the first direction.

2. The surgical instrument of claim 1, wherein the second guide mechanism is located at a proximal end or a distal end of the first guide mechanism.

3. The surgical instrument of claim 2, wherein the first segment cable bundle is substantially perpendicular to cable segments of the plurality of cables between the plurality of drive units and the first guide mechanism.

4. The surgical instrument of claim 2, wherein the proximal end of the elongate shaft is located at an edge of the drive device.

5. The surgical instrument of claim 2, wherein the first direction is non-parallel with the second direction.

6. The surgical instrument of claim 1, wherein the drive device further comprises a third guide mechanism located near the proximal end of the elongate shaft, the second segment cable bundle is located between the second guide mechanism and the third guide mechanism, the cable bundle further comprises a third segment cable bundle arranged between the third guide mechanism and the proximal end of the elongate shaft, and the third segment cable bundle extends in the elongate shaft in a third direction opposite to the first direction.

7. The surgical instrument of claim 6, wherein the first guide mechanism comprises a plurality of first pulleys, each of the first pulleys comprises a first axle and a first guide part for guiding one of the plurality of cables, the first guide part is rotatably mounted on the first axle, and the first axles of the plurality of first pulleys are substantially perpendicular to rotation axes of the plurality of drive units.

8. The surgical instrument of claim 7, wherein the second guide mechanism comprises a plurality of second pulleys, each of the second pulleys comprises a second axle and a second guide part for guiding one cable segment of the first segment cable bundle, the second guide part is rotatably mounted on the second axle, and the second axles of the plurality of second pulleys are substantially perpendicular to rotation axles of the plurality of drive units.

9. The surgical instrument of claim 8, wherein the third guide mechanism comprises a plurality of third pulleys, each of the third pulleys comprises a third axle and a third guide part for guiding one cable segment of the second segment cable bundle, the third guide part is rotatably mounted on the third axle, and the third axles of the plurality of third pulleys are substantially perpendicular to rotation axles of the plurality of drive units.

10. The surgical instrument of claim 8, wherein the plurality of cables comprises a first cable, an acute angel is formed between the first axle of the pulley that guides the first cable among the plurality of first pulleys and the second axle of the pulley that guides the first cable among the plurality of second pulleys.

11. The surgical instrument of claim 10, wherein the plurality of cables further comprises a second cable, and the first axle of the pulley that guides the second cable among the plurality of first pulleys is substantially perpendicular to the second axle of the pulley that guides the second cable among the plurality of second pulleys.

12. The surgical instrument of claim 11, wherein the plurality of cables further comprises a third cable, and the first axle of the pulley that guides the third cable among the plurality of the first pulleys is substantially parallel to the second axle of the pulley that guides the third cable among the plurality of the second pulleys.

13. The surgical instrument of claim 12, wherein the cable segment of the first cable between the plurality of drive units and the first guide mechanism and the cable segment of the first cable in the first segment cable bundle are on a first plane; the cable segment of the first cable in the first segment cable bundle and the cable segment of the first cable in the second segment cable bundle are on a second plane, wherein the first plane intersects with the second plane.

14. The surgical instrument of claim 12, wherein the cable segment of the second cable between the plurality of drive units and the first guide mechanism and the cable segment of the second cable in the first segment cable bundle are on a third plane; the cable segment of the first cable in the first segment cable bundle and the cable segment of the first cable in the second segment cable bundle are on a fourth plane, wherein the third plane is substantially perpendicular to the fourth plane.

15. The surgical instrument of claim 12, wherein the cable segment of the third cable between the plurality of drive units and the first guide mechanism and the cable segment of the third cable in the first segment cable bundle are on a fifth plane; the cable segment of the third cables in the first segment cable bundle and the cable segment of the third cables in the second segment cable bundle are on a sixth plane, wherein the fifth plane is substantially parallel to the sixth plane.

16. The surgical instrument of claim 1, wherein the plurality of cables comprise a plurality of first cable segments formed between the plurality of drive units and the first guide mechanism, and a lengthwise direction of any cable segment of the plurality of first cable segments is substantially perpendicular to the lengthwise direction of the first segment cable bundle.

17. The surgical instrument of claim 16, wherein the cable segments of the plurality of cables in the first segment cable bundle are substantially parallel to each other.

18. The surgical instrument of claim 1, wherein a lengthwise direction of the second segment cable bundle is substantially perpendicular to a lengthwise direction of the first segment cable bundle.

19. The surgical instrument of claim 18, wherein cable segments of at least two of the plurality of cables in the second segment cable bundle are substantially parallel to each other.

20. The surgical instrument of claim 6, wherein a lengthwise direction of the third segment cable bundle is substantially parallel to a lengthwise direction of the first segment cable bundle.

21. The surgical instrument of claim 20, wherein the lengthwise direction of the third segment cable bundle is substantially perpendicular to a lengthwise direction of the second segment cable bundle.

22. The surgical instrument of claim 21, wherein the cable segments of the plurality of cables in the third segment cable bundle are substantially parallel to each other.

23. The surgical instrument of claim 7, wherein at least one of the plurality of first pulleys of the first guide mechanism is adjustable to lie on different levels within the drive device.

24. The surgical instrument of claim 8, wherein at least one of the plurality of second pulleys of the second guide mechanism is adjustable to lie on different levels within the device.

25. The surgical instrument of claim 1, wherein the plurality of drive units are located on vertices of a polygon.

26. The surgical instrument of claim 1, wherein the first guide mechanism is located in a middle area of the plurality of drive units.

27. The surgical instrument of claim 1, wherein the first guide mechanism and/or the second guide mechanism comprise conduits for guiding the plurality of cables.

28. A slave operating equipment, comprising a mechanical arm and the surgical instrument according to any one of claims 1-27, the surgical instrument is mounted on the mechanical arm, and the mechanical arm is configured to control the movement of the surgical instrument.

29. A surgical robot, the surgical robot comprises a master operating equipment and the slave operating equipment according to claim 28, and the slave operating equipment performs corresponding operations according to the instructions of the master operating equipment.
